(19) Europäisches Patentamt
European Patent Office
Office européen des brevets

(11) Veröffentlichungsnummer: **0 191 282**
**B1**

(12) **EUROPÄISCHE PATENTSCHRIFT**

(45) Veröffentlichungstag der Patentschrift: **04.10.89**

(51) Int. Cl.⁴: **C 07 D 231/16**, A 01 N 43/56

(21) Anmeldenummer: **86100093.3**

(22) Anmeldetag: **07.01.86**

(54) **1-Aryl-4-Nitropyrazole.**

(30) Priorität: **17.01.85 DE 3501323**

(43) Veröffentlichungstag der Anmeldung: **20.08.86 Patentblatt 86/34**

(45) Bekanntmachung des Hinweises auf die Patenterteilung: **04.10.89 Patentblatt 89/40**

(84) Benannte Vertragsstaaten: **AT BE CH DE FR GB IT LI NL SE**

(56) Entgegenhaltungen:
**CH-A-573 919**
**US-A-3 869 274**

**Die Akte enthält technische Angaben, die nach dem Eingang der Anmeldung eingereicht wurden und die nicht in dieser Patentschrift enthalten sind.**

(73) Patentinhaber: **BAYER AG, D-5090 Leverkusen 1 Bayerwerk (DE)**

(72) Erfinder: **Gehring, Reinhold, Dr., Dasnoeckel 49, D-5600 Wuppertal 11 (DE)**
Erfinder: **Schallner, Otto, Dr., Noldeweg 22, D-4019 Monheim (DE)**
Erfinder: **Stetter, Jörg, Dr., Gellertweg 4, D-5600 Wuppertal 1 (DE)**
Erfinder: **Santel, Hans- Joachim, Dr., Grünstrasse 9a, D-5090 Leverkusen 1 (DE)**
Erfinder: **Schmidt, Robert R., Dr., Im Waldwinkel 110, D-5060 Bergisch- Gladbach 2 (DE)**



Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft neue 1-Aryl-4-nitropyrazole, mehrere Verfahren zu ihrer Herstellung und ihre Verwendung als Herbizide.

Es ist bereits bekannt, daß bestimmte 1-Aryl-pyrazole, wie beispielsweise das 4-Cyano-5-propionamido-1-(2,3,4-trichlorphenyl)-pyrazol, herbizide Eigenschaften besitzen (vgl. z. B. DE-OS-3 226 513).

Die herbizide Wirksamkeit dieser vorbekannten Verbindungen gegenüber Unkräutern ist jedoch ebenso wie ihre Verträglichkeit gegenüber wichtigen Kulturpflanzen nicht immer in allen Anwendungsgebieten völlig zufriedenstellend.

Es wurden nun neue 1-Aryl-4-nitropyrazole der allgemeinen Formel (I),

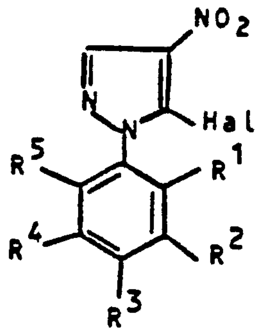

(I)

in welcher

Hal für Halogen steht,

$R^1$ und $R^3$ unabhängig voneinander für Cyano, Nitro, Halogen oder für jeweils geradkettiges oder verzweigtes Alkyl, Alkoxy oder Alkoxycarbonyl mit jeweils 1 bis 4 Kohlenstoffatomen im Alkylteil, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest $-S(O)_n-R^6$ stehen, und

$R^2$, $R^4$ und $R^5$ unabhängig voneinander und unabhängig von $R^1$ und $R^3$ für die gleichen Reste wie $R^1$ und $R^3$ und zusätzlich für Wasserstoff stehen, wobei $R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

n für eine Zahl 0, 1 oder 2 steht,

gefunden.

Weiterhin wurde gefunden, daß man die neuen 1-Aryl-4-nitropyrazole der allgemeinen Formel (I),

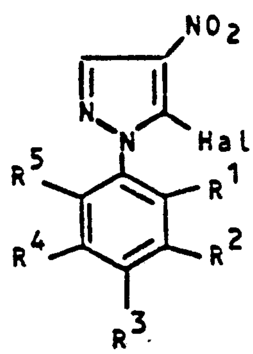

(I)

in welcher

Hal, $R^1$, $R^2$, $R^3$, $R^4$ und $R^5$ die oben angegebenen Bedeutungen haben erhält, wenn man

(1a) 1-Arylpyrazole der allgemeinen Formel (II),

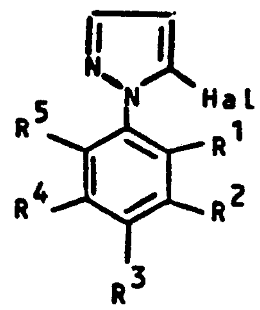

(II)

in welcher

$R^1$, $R^2$, $R^3$, $R^4$, $R^5$ und Hal die oben angegebene Bedeutung haben,

mit Salpetersäure gegebenenfalls in Gegenwart eines Verdünnungsmittels, sowie gegebenenfalls in Gegenwart eines Katalysators umsetzt, oder wenn man

(b) 5-Amino-4-nitropyrazole der Formel (III),

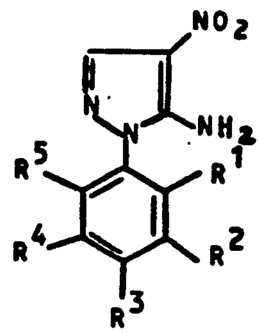

(III)

in welcher

$R^1$, $R^2$, $R^3$, $R^4$, $R^5$ und Hal die oben angegebene Bedeutung haben,

mit Nitritverbindungen der Formel (IV),

R-O-N=O (IV)

in welcher

R für Wasserstoff, ein Alkalimetallkation oder für Alkyl steht,

und Haloform der Formel (V),

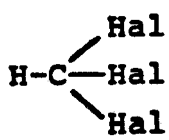

(V)

in welcher

Hal die oben angegebene Bedeutung hat, gegebenenfalls in Gegenwart eines Katalysators umsetzt, oder wenn man

(c) die nach Verfahren (a) oder (b) erhältlichen 1-Aryl-4-nitropyrazole der Formel (I)

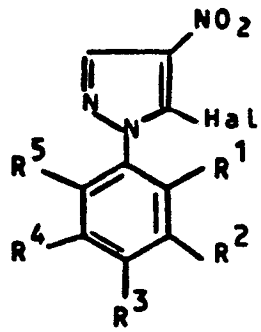

(I)

in welcher
$R^1$, $R^2$, $R^3$, $R^4$, $R^5$ und Hal die oben angegebene Bedeutung haben,
mit Halogeniden der Formel (VI),

$M^{\oplus}$ $Hal'^{\ominus}$ (VI)

in welcher
$M^{\oplus}$ für ein Äquivalent eines Metallkations oder für ein gegebenenfalls substituiertes Ammoniumion steht und
$Hal'^{\ominus}$ für Halogen steht, aber verschieden von Hal ist,
gegebenenfalls in Gegenwart eines Verdünnungsmittels und gegebenenfalls in Gegenwart eines Phasentransfer-Katalysators umsetzt.

Auf diese Weise erhält man aus einem bestimmten Halogenderivat der 1-Aryl-4-nitropyrazole der Formel (I), z. B. dem Chlorderivat, durch Umhalogenierung, z. B. mit Natriumjodid, die entsprechenden Halogenderivate der 1-Aryl-4-nitropyrazole der Formel (I), in denen z. B. Chlor durch Jod ersetzt ist.

Schließlich wurde gefunden, daß die neuen 1-Aryl-4-nitropyrazole der allgemeinen Formel (I) herbizide, insbesondere auch selektiv-herbizide Eigenschaften besitzen.

Überraschenderweise zeigen die erfindungsgemäßen 1-Aryl-4-nitropyrazole der allgemeinen Formel (I) bei einer vergleichbaren allgemein herbiziden Wirksamkeit gegen schwer bekämpfbare Unkräuter eine erheblich verbesserte Kulturpflanzenselektivität im Vergleich zu den aus dem Stand der Technik bekannten 1-Arylpyrazolen, wie beispielsweise dem 4-Cyano-5-propionylamino-1-(2, 3,4-trichlor-phenyl)-pyrazol, welches chemisch und wirkungsmäßig naheliegende Verbindungen sind.

Die erfindungsgemäßen 1-Aryl-4-nitropyrazole sind durch die Formel (I) allgemein definiert. Bevorzugt sind Verbindungen der Formel (I), bei welchen
Hal für Fluor, Chlor, Brom oder Iod steht,
$R^1$ und $R^3$ unabhängig voneinander für Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^6$ stehen und
$R^2$, $R^4$ und $R^5$ unabhängig voneinander und unabhängig von $R^1$ und $R^3$ für die gleichen Reste wie $R^1$ und $R^3$ und zusätzlich für Wasserstoff stehen, wobei
$R^6$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht
und
n für eine Zahl 0, 1 oder 2 steht.

Im einzelnen seien außer den bei den Herstellungsbeispielen genannten Verbindungen die in der folgenden Tabelle formelmäßig aufgeführten 1-Aryl-4-nitropyrazole der allgemeinen Formel (I) genannt:

**Tabelle 1**

| Hal | $R^1$ | $R^2$ | $R^3$ | $R^4$ | $R^5$ |
|---|---|---|---|---|---|
| Cl | Cl | H | Cl | H | H |
| Cl | Cl | Cl | Cl | H | H |
| Cl | Cl | H | Cl | H | Cl |
| Cl | $NO_2$ | H | $NO_2$ | H | H |
| Cl | Cl | H | $CF_3$ | H | H |
| Cl | Cl | H | $CF_3$ | H | Cl |
| Cl | $CF_3$ | H | $CF_3$ | H | H |

| | | | | | |
|---|---|---|---|---|---|
| Cl | Cl | Cl | $CF_3$ | H | Cl |
| Cl | Cl | Cl | Cl | H | Cl |
| Cl | Cl | H | $OCF_3$ | H | H |
| Cl | Cl | H | $OCF_3$ | H | Cl |
| Cl | Cl | H | $SCF_3$ | H | H |
| Cl | Cl | H | $SCF_3$ | H | Cl |
| Cl | Cl | Cl | $SCF_3$ | H | H |
| Cl | Cl | Cl | $SCF_3$ | H | Cl |
| Cl | F | F | $OCF_3$ | H | F |
| Cl | F | F | $OCF_3$ | F | F |
| Cl | Cl | Cl | $OCF_3$ | H | H |
| Cl | Br | H | $SCF_3$ | H | H |
| Cl | Br | H | $OCF_3$ | H | Br |
| Cl | $NO_2$ | H | $NO_2$ | H | $NO_2$ |
| Cl | F | H | $OCF_3$ | H | F |
| Cl | F | H | $CF_3$ | H | F |
| Cl | F | F | $SCF_3$ | H | F |
| Cl | F | H | $SCF_3$ | H | F |
| Cl | I | H | $OCF_3$ | H | H |
| Cl | Cl | H | $SO_2CF_3$ | H | Cl |
| Cl | $CF_3$ | H | $CF_3$ | H | $CF_3$ |
| Cl | $CF_3$ | H | $SO_2CF_3$ | H | $CF_3$ |
| Cl | $CF_3$ | H | $SCF_3$ | H | $CF_3$ |
| Cl | $CF_3$ | H | $SCF_3$ | H | H |
| Cl | Br | H | $CF_3$ | H | Br |
| Cl | F | F | $SO_2CF_3$ | H | F |
| Cl | Cl | Cl | $SCF_3$ | Cl | Cl |
| Cl | Cl | F | Cl | H | Cl |
| Cl | F | F | F | F | F |
| Cl | F | F | $CF_3$ | F | F |
| Cl | Cl | Cl | Cl | Cl | Cl |
| Cl | Cl | H | $SO_2CH_3$ | H | Cl |
| Cl | Cl | Cl | $SO_2CF_3$ | H | H |
| Cl | Br | H | $SO_2CF_3$ | H | Br |
| Cl | $NO_2$ | H | $CF_3$ | H | H |
| Cl | F | H | $SO_2CF_3$ | H | F |
| Cl | Cl | H | $OCH_3$ | H | Cl |
| Cl | Cl | H | $SCH_3$ | H | Cl |
| Cl | F | H | F | H | F |
| Cl | Cl | H | $SO_2CH_3$ | H | H |
| Cl | Cl | H | $SO_2CH_3$ | H | Cl |
| Cl | Cl | H | $SO_2CClF_2$ | H | Cl |
| Cl | Br | H | $OCF_3$ | H | H |
| Cl | Br | H | $SCF_3$ | H | Br |
| Cl | Br | H | Br | H | Br |
| Cl | Br | H | Br | H | H |
| Cl | Br | H | Cl | H | Cl |
| Cl | Cl | H | Br | H | Cl |
| Cl | Cl | H | Br | H | Br |
| Cl | Cl | H | $SO_2CF_3$ | H | H |
| Cl | Cl | H | $SO_2CF_2Cl$ | H | H |
| Cl | Cl | H | $SO_2CF_2Cl$ | H | Cl |
| Cl | Br | H | $SO_2CF_2Cl$ | H | H |
| Cl | Cl | H | $OCF_2Cl$ | H | H |
| Cl | Cl | H | $OCF_2Cl$ | H | Cl |
| Cl | Cl | H | $OCF_2CHF_2$ | H | Cl |
| Cl | Cl | H | $CF_2Cl$ | H | Cl |
| Cl | Cl | H | $OCF_2Cl$ | H | H |
| Cl | Cl | H | $OCHF_2$ | H | H |
| Cl | Cl | H | $OCHF_2$ | H | Cl |
| Cl | Cl | H | $OCH_2CF_3$ | H | H |
| Cl | Cl | H | $OCH_2CF_3$ | H | Cl |
| Cl | Cl | H | $OCF_2CHFCl$ | H | H |
| Cl | Cl | H | $OCF_2CHFCl$ | H | Cl |
| Cl | Cl | H | $CHF_2$ | H | Cl |

| | | | | | |
|---|---|---|---|---|---|
| Cl | Cl | H | $CHF_2$ | H | H |
| Cl | Br | H | $OCH_2CF_3$ | H | Br |
| Cl | Br | H | $OCH_2CF_3$ | H | H |
| Cl | Br | H | $SO_2CF_2Cl$ | H | H |
| Cl | Br | H | $SO_2CF_2Cl$ | H | Br |
| Cl | Br | H | $SO_2CH_3$ | H | H |
| Cl | Br | H | $SO_2CH_3$ | H | Br |
| Br | Cl | H | Cl | H | H |
| Br | Cl | Cl | Cl | H | H |
| Br | Cl | H | Cl | H | Cl |
| Br | $NO_2$ | H | $NO_2$ | H | H |
| Br | Cl | H | $CF_3$ | H | H |
| Br | Cl | H | $CF_3$ | H | Cl |
| Br | $CF_3$ | H | $CF_3$ | H | H |
| Br | Cl | Cl | $CF_3$ | H | Cl |
| Br | Cl | Cl | Cl | H | Cl |
| Br | Cl | H | $OCF_3$ | H | H |
| Br | Cl | H | $OCF_3$ | H | Cl |
| Br | Cl | H | $SCF_3$ | H | H |
| Br | Cl | H | $SCF_3$ | H | Cl |
| Br | Cl | Cl | $SCF_3$ | H | H |
| Br | Cl | Cl | $SCF_3$ | H | Cl |
| Br | F | F | $OCF_3$ | H | F |
| Br | F | F | $OCF_3$ | F | F |
| Br | Cl | Cl | $OCF_3$ | H | H |
| Br | Br | H | $SCF_3$ | H | H |
| Br | Br | H | $OCF_3$ | H | Br |
| Br | $NO_2$ | H | $NO_2$ | H | $NO_2$ |
| Br | F | H | $OCF_3$ | H | F |
| Br | F | H | $CF_3$ | H | F |
| Br | F | F | $SCF_3$ | H | F |
| Br | F | H | $SCF_3$ | H | F |
| Br | I | H | $OCF_3$ | H | H |
| Br | Cl | H | $SO_2CF_3$ | H | Cl |
| Br | $CF_3$ | H | $CF_3$ | H | $CF_3$ |
| Br | $CF_3$ | H | $SO_2CF_3$ | H | $CF_3$ |
| Br | $CF_3$ | H | $SCF_3$ | H | $CF_3$ |
| Br | $CF_3$ | H | $SCF_3$ | H | H |
| Br | Br | H | $CF_3$ | H | Br |
| Br | F | F | $SO_2CF_3$ | H | F |
| Br | Cl | Cl | $SCF_3$ | Cl | Cl |
| Br | Cl | F | Cl | H | Cl |
| Br | F | F | F | F | F |
| Br | F | F | $CF_3$ | F | F |
| Br | Cl | Cl | Cl | Cl | Cl |
| Br | Cl | H | $SO_2CH_3$ | H | Cl |
| Br | Cl | Cl | $SO_2CF_3$ | H | H |
| Br | Br | H | $SO_2CF_3$ | H | Br |
| Br | $NO_2$ | H | $CF_3$ | H | H |
| Br | F | H | $SO_2CF_3$ | H | F |
| Br | Cl | H | $OCH_3$ | H | Cl |
| Br | Cl | H | $SCH_3$ | H | Cl |
| Br | F | H | F | H | F |
| Br | Cl | H | $SO_2CH_3$ | H | H |
| Br | Cl | H | $SO_2CH_3$ | H | Cl |
| Br | Cl | H | $SO_2CClF_2$ | H | Cl |
| Br | Br | H | $OCF_3$ | H | H |
| Br | Br | H | $SCF_3$ | H | Br |
| Br | Br | H | Br | H | Br |
| Br | Br | H | Br | H | H |
| Br | Br | H | Cl | H | Cl |
| Br | Cl | H | Br | H | Cl |
| Br | Cl | H | Br | H | Br |
| Br | Cl | H | $SO_2CF_3$ | H | H |

| | | | | | |
|---|---|---|---|---|---|
| Br | Cl | H | $SO_2CF_2Cl$ | H | H |
| Br | Cl | H | $SO_2CF_2Cl$ | H | Cl |
| Br | Br | H | $SO_2CF_2Cl$ | H | H |
| Br | Cl | H | $OCF_2Cl$ | H | H |
| Br | Cl | H | $OCF_2Cl$ | H | Cl |
| Br | Cl | H | $OCF_2CHF_2$ | H | Cl |
| Br | Cl | H | $CF_2Cl$ | H | Cl |
| Br | Cl | H | $OCF_2Cl$ | H | H |
| Br | Cl | H | $OCHF_2$ | H | H |
| Br | Cl | H | $OCHF_2$ | H | Cl |
| Br | Cl | H | $OCH_2CF_3$ | H | H |
| Br | Cl | H | $OCH_2CF_3$ | H | Cl |
| Br | Cl | H | $OCF_2CHFCl$ | H | H |
| Br | Cl | H | $OCF_2CHFCl$ | H | Cl |
| Br | Cl | H | $CHF_2$ | H | Cl |
| Br | Cl | H | $CHF_2$ | H | H |
| Br | Br | H | $OCH_2CF_3$ | H | Br |
| Br | Br | H | $OCH_2CF_3$ | H | H |
| Br | Br | H | $SO_2CF_2Cl$ | H | H |
| Br | Br | H | $SO_2CF_2Cl$ | H | Br |
| Br | Br | H | $SO_2CH_3$ | H | H |
| Br | Br | H | $SO_2CH_3$ | H | Br |
| F | Cl | H | Cl | H | H |
| F | Cl | Cl | Cl | H | H |
| F | Cl | H | Cl | H | Cl |
| F | $NO_2$ | H | $NO_2$ | H | H |
| F | Cl | H | $CF_3$ | H | H |
| F | Cl | H | $CF_3$ | H | Cl |
| F | $CF_3$ | H | $CF_3$ | H | H |
| F | Cl | Cl | $CF_3$ | H | Cl |
| F | Cl | Cl | Cl | H | Cl |
| F | Cl | H | $OCF_3$ | H | H |
| F | Cl | H | $OCF_3$ | H | Cl |
| F | Cl | H | $SCF_3$ | H | H |
| F | Cl | H | $SCF_3$ | H | Cl |
| F | Cl | Cl | $SCF_3$ | H | H |
| F | Cl | Cl | $SCF_3$ | H | Cl |
| F | F | F | $OCF_3$ | H | F |
| F | F | F | $OCF_3$ | F | F |
| F | Cl | Cl | $OCF_3$ | H | H |
| F | Br | H | $SCF_3$ | H | H |
| F | Br | H | $OCF_3$ | H | Br |
| F | $NO_2$ | H | $NO_2$ | H | $NO_2$ |
| F | F | H | $OCF_3$ | H | F |
| F | F | H | $CF_3$ | H | F |
| F | F | F | $SCF_3$ | H | F |
| F | F | H | $SCF_3$ | H | F |
| F | I | H | $OCF_3$ | H | H |
| F | Cl | H | $SO_2CF_3$ | H | Cl |
| F | $CF_3$ | H | $CF_3$ | H | $CF_3$ |
| F | $CF_3$ | H | $SO_2CF_3$ | H | $CF_3$ |
| F | $CF_3$ | H | $SCF_3$ | H | $CF_3$ |
| F | $CF_3$ | H | $SCF_3$ | H | H |
| F | Br | H | $CF_3$ | H | Br |
| F | F | F | $SO_2CF_3$ | H | F |
| F | Cl | Cl | $SCF_3$ | Cl | Cl |
| F | Cl | F | Cl | H | Cl |
| F | F | F | F | F | F |
| F | F | F | $CF_3$ | F | F |
| F | Cl | Cl | Cl | Cl | Cl |
| F | Cl | H | $SO_2CH_3$ | H | Cl |
| F | Cl | Cl | $SO_2CF_3$ | H | H |
| F | Br | H | $SO_2CF_3$ | H | Br |
| F | $NO_2$ | H | $CF_3$ | H | H |

| | | | | | |
|---|---|---|---|---|---|
| F | F | H | $SO_2CF_3$ | H | F |
| F | Cl | H | $OCH_3$ | H | Cl |
| F | Cl | H | $SCH_3$ | H | Cl |
| F | F | H | F | H | F |
| F | Cl | H | $SO_2CH_3$ | H | H |
| F | Cl | H | $SO_2CH_3$ | H | Cl |
| F | Cl | H | $SO_2CClF_2$ | H | Cl |
| F | Br | H | $OCF_3$ | H | H |
| F | Br | H | $SCF_3$ | H | Br |
| F | Br | H | Br | H | Br |
| F | Br | H | Br | H | H |
| F | Br | H | Cl | H | Cl |
| F | Cl | H | Br | H | Cl |
| F | Cl | H | Br | H | Br |
| F | Cl | H | $SO_2CF_3$ | H | H |
| F | Cl | H | $SO_2CF_2Cl$ | H | H |
| F | Cl | H | $SO_2CF_2Cl$ | H | Cl |
| F | Br | H | $SO_2CF_2Cl$ | H | H |
| F | Cl | H | $OCF_2Cl$ | H | H |
| F | Cl | H | $OCF_2Cl$ | H | Cl |
| F | Cl | H | $OCF_2CHF_2$ | H | Cl |
| F | Cl | H | $CF_2Cl$ | H | Cl |
| F | Cl | H | $OCF_2Cl$ | H | H |
| F | Cl | H | $OCHF_2$ | H | H |
| F | Cl | H | $OCHF_2$ | H | Cl |
| F | Cl | H | $OCH_2CF_3$ | H | H |
| F | Cl | H | $OCH_2CF_3$ | H | Cl |
| F | Cl | H | $OCF_2CHFCl$ | H | H |
| F | Cl | H | $OCF_2CHFCl$ | H | Cl |
| F | Cl | H | $CHF_2$ | H | Cl |
| F | Cl | H | $CHF_2$ | H | H |
| F | Br | H | $OCH_2CF_3$ | H | Br |
| F | Br | H | $OCH_2CF_3$ | H | H |
| F | Br | H | $SO_2CF_2Cl$ | H | H |
| F | Br | H | $SO_2CF_2Cl$ | H | Br |
| F | Br | H | $SO_2CH_3$ | H | H |
| F | Br | H | $SO_2CH_3$ | H | Br |
| I | Cl | H | Cl | H | H |
| I | Cl | Cl | Cl | H | H |
| I | Cl | H | Cl | H | Cl |
| I | $NO_2$ | H | $NO_2$ | H | H |
| I | Cl | H | $CF_3$ | H | H |
| I | Cl | H | $CF_3$ | H | Cl |
| I | $CF_3$ | H | $CF_3$ | H | H |
| I | Cl | Cl | $CF_3$ | H | Cl |
| I | Cl | Cl | Cl | H | Cl |
| I | Cl | H | $OCF_3$ | H | H |
| I | Cl | H | $OCF_3$ | H | Cl |
| I | Cl | H | $SCF_3$ | H | H |
| I | Cl | H | $SCF_3$ | H | Cl |
| I | Cl | Cl | $SCF_3$ | H | H |
| I | Cl | Cl | $SCF_3$ | H | Cl |
| I | F | F | $OCF_3$ | H | F |
| I | F | F | $OCF_3$ | F | F |
| I | Cl | Cl | $OCF_3$ | H | H |
| I | Br | H | $SCF_3$ | H | H |
| I | Br | H | $OCF_3$ | H | Br |
| I | $NO_2$ | H | $NO_2$ | H | $NO_2$ |
| I | F | H | $OCF_3$ | H | F |
| I | F | H | $CF_3$ | H | F |
| I | F | F | $SCF_3$ | H | F |
| I | F | H | $SCF_3$ | H | F |
| I | I | H | $OCF_3$ | H | H |
| I | Cl | H | $SO_2CF_3$ | H | Cl |

| | | | | | |
|---|---|---|---|---|---|
| I | $CF_3$ | H | $CF_3$ | H | $CF_3$ |
| I | $CF_3$ | H | $SO_2CF_3$ | H | $CF_3$ |
| I | $CF_3$ | H | $SCF_3$ | H | $CF_3$ |
| I | $CF_3$ | H | $SCF_3$ | H | H |
| I | Br | H | $CF_3$ | H | Br |
| I | F | F | $SO_2CF_3$ | H | F |
| I | Cl | Cl | $SCF_3$ | Cl | Cl |
| I | Cl | F | Cl | H | Cl |
| I | F | F | F | F | F |
| I | F | F | $CF_3$ | F | F |
| I | Cl | Cl | Cl | Cl | Cl |
| I | Cl | H | $SO_2CH_3$ | H | Cl |
| I | Cl | Cl | $SO_2CF_3$ | H | H |
| I | Br | H | $SO_2CF_3$ | H | Br |
| I | $NO_2$ | H | $CF_3$ | H | H |
| I | F | H | $SO_2CF_3$ | H | F |
| I | Cl | H | $OCH_3$ | H | Cl |
| I | Cl | H | $SCH_3$ | H | Cl |
| I | F | H | F | H | F |
| I | Cl | H | $SO_2CH_3$ | H | H |
| I | Cl | H | $SO_2CH_3$ | H | Cl |
| I | Cl | H | $SO_2CClF_2$ | H | Cl |
| I | Br | H | $OCF_3$ | H | H |
| I | Br | H | $SCF_3$ | H | Br |
| I | Br | H | Br | H | Br |
| I | Br | H | Br | H | H |
| I | Br | H | Cl | H | Cl |
| I | Cl | H | Br | H | Cl |
| I | Cl | H | Br | H | Br |
| I | Cl | H | $SO_2CF_3$ | H | H |
| I | Cl | H | $SO_2CF_2Cl$ | H | H |
| I | Cl | H | $SO_2CF_2Cl$ | H | Cl |
| I | Br | H | $SO_2CF_2Cl$ | H | H |
| I | Cl | H | $OCF_2Cl$ | H | H |
| I | Cl | H | $OCF_2Cl$ | H | Cl |
| I | Cl | H | $OCF_2CHF_2$ | H | Cl |
| I | Cl | H | $CF_2Cl$ | H | Cl |
| I | Cl | H | $OCF_2Cl$ | H | H |
| I | Cl | H | $OCHF_2$ | H | H |
| I | Cl | H | $OCHF_2$ | H | Cl |
| I | Cl | H | $OCH_2CF_3$ | H | H |
| I | Cl | H | $OCH_2CF_3$ | H | Cl |
| I | Cl | H | $OCF_2CHFCl$ | H | H |
| I | Cl | H | $OCF_2CHFCl$ | H | Cl |
| I | Cl | H | $CHF_2$ | H | Cl |
| I | Cl | H | $CHF_2$ | H | H |
| I | Br | H | $OCH_2CF_3$ | H | Br |
| I | Br | H | $OCH_2CF_3$ | H | H |
| I | Br | H | $SO_2CF_2Cl$ | H | H |
| I | Br | H | $SO_2CF_2Cl$ | H | Br |
| I | Br | H | $SO_2CH_3$ | H | H |
| I | Br | H | $SO_2CH_3$ | H | Br |

Verwendet man beispielsweise 5-Chlor-1-(2,4,6-trichlorphenyl)-pyrazol und Salpetersäure als Ausgangsstoffe, so läßt sich der Reaktionsablauf des erfindungsgemäßen Verfahrens (a) durch das folgende Formelschema darstellen:

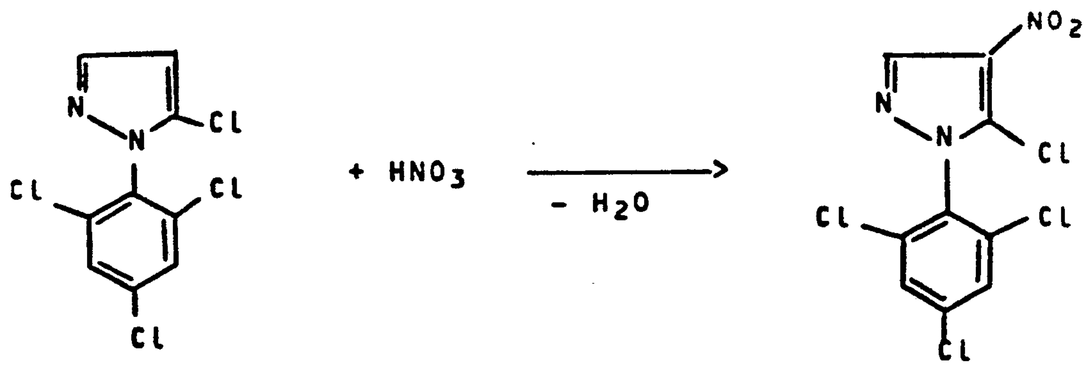


Verwendet man beispielsweise 5-Amino-4-nitro-1-(2,4,6-trichlorphenyl)-pyrazol, t-Butylnitrit und Bromoform als Ausgangsstoffe, so läßt sich der Reaktionsablauf des erfindungsgemäßen Verfahrens (b) durch das folgende Formelschema darstellen:

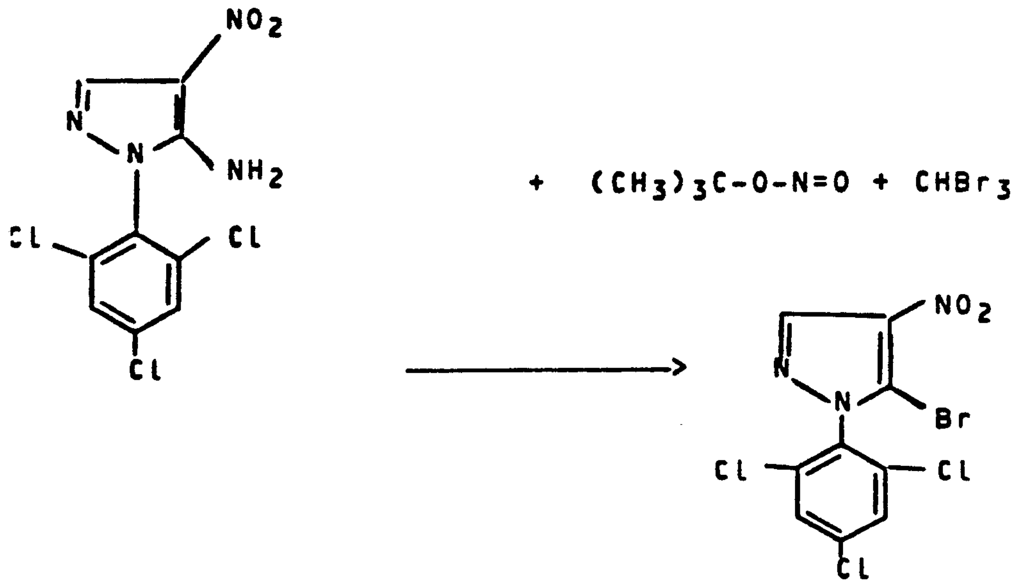


Verwendet man beispielsweise 5-Chlor-4-nitro-1-(2,3,4-trichlorphenyl)-pyrazol und Natriumiodid als Ausgangsstoffe, so läßt sich der Reaktionsablauf des erfindungsgemäßen Verfahrens (c) durch das folgende Formelschema darstellen:

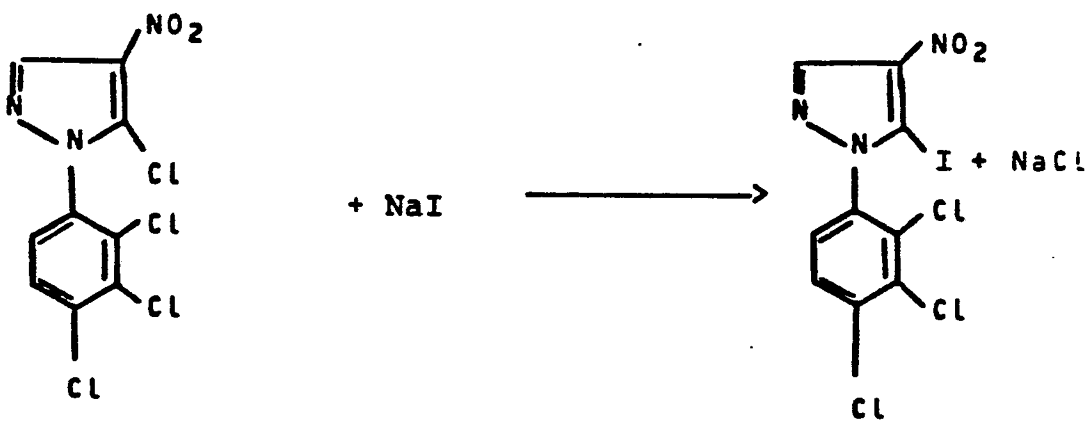


Die zur Durchführung des erfindungsgemäßen Verfahrens (a) als Ausgangsstoffe benötigten 1-Arylpyrazole sind durch die Formel (II) allgemein definiert. In dieser Formel (II) stehen $R^1$, $R^2$, $R^3$, $R^4$, $R^5$ und Hal vorzugsweise für diejenigen Reste, die bereits im Zusammenhang mit der Beschreibung der erfindungsgemäßen Stoffe der Formel (I) als bevorzugt für diese Substituenten genannt wurden.

Die 1-Arylpyrazole der Formel (II) sind noch nicht bekannt. Man erhält sie, wenn man Alkoxymethylenmalonester der Formel (VII),

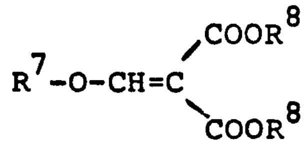

(VII)

in welcher

$R^7$ und $R^8$ unabhängig voneinander jeweils für Alkyl, insbesondere für Methyl oder Ethyl stehen, mit Phenylhydrazinen der Formel (VIII),

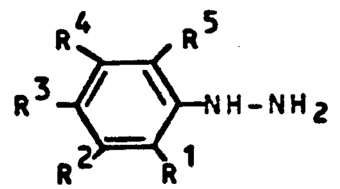

(VIII)

in welcher

$R^1$, $R^2$, $R^3$, $R^4$ und $R^5$ die oben angegebene Bedeutung haben,

zunächst in einer ersten Stufe gegebenenfalls in Gegenwart eines Verdünnungsmittels wie beispielsweise Methanol oder Ethanol bei Temperaturen zwischen +10°C und +80°C umsetzt.

Das hierbei intermediär auftretende Zwischenprodukt der Formel (VIIIa),

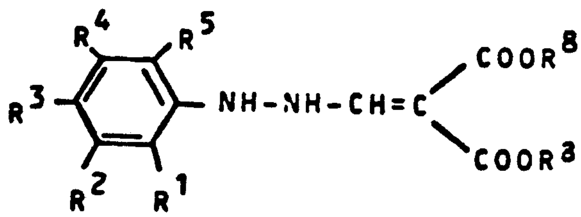

(VIIIa)

in welcher

$R^1$, $R^2$, $R^3$, $R^4$, $R^5$ und $R^8$ die oben angegebene Bedeutung haben,

kann gegebenenfalls auch isoliert und in einer separaten Reaktionsstufe cyclisiert werden.

Die so erhältlichen Pyrazolcarbonsäureester der Formel (IX),

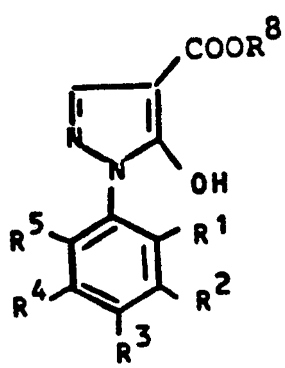

(IX)

in welcher

$R^1$, $R^2$, $R^3$, $R^4$, $R^5$ und $R^8$ die oben angegebene Bedeutung haben

werden in einer 2. Stufe gegebenenfalls in Gegenwart eines Verdünnungsmittels wie beispielsweise Methanol und gegebenenfalls in Gegenwart einer Base wie beispielsweise Natriumhydroxid bei Temperaturen zwischen 30°C und 70°C decarboxyliert und in einer 3. Stufe werden die so erhältlichen Pyrazoline der Formel (X),

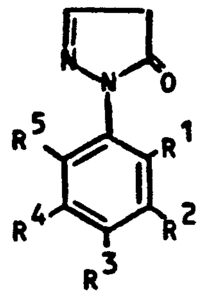

(X)

in welcher

$R^1$, $R^2$, $R^3$, $R^4$ und $R^5$ die oben angegebene Bedeutung haben,

mit Halogenierungsmitteln, wie beispielsweise Phosphoroxychlorid oder Phosphoroxybromid nach üblichen, bekannten Verfahren (vgl. z. B. Ber. dtsch. chem. Ges. 28, 35 [1895] oder Liebigs Ann. Chem. 373, 129 [1910]) umsetzt.

Die Cyclisierung und anschließende Decarboxylierung können gegebenenfalls auch in einer Reaktionsstufe als "Eintopfverfahren" durchgeführt werden (vgl. z. B. Liebigs Ann. Chem. 373, 142 [1910] und die Herstellungsbeispiele).

Die Alkoxymethylenmalonester der Formel (VII) sind allgemein bekannte Verbindungen der organischen Chemie.

Die Phenylhydrazine der Formel (VIII) sind größtenteils bekannt oder können nach bekannten Verfahren in einfacher, analoger Weise hergestellt werden (vgl. z. B. Houben-Weyl "Methoden der organischen Chemie" Band X, 2, S. 203, Thieme Verlag, Stuttgart 1967), indem man beispielsweise die bekannten Aniline der Formel (XI)

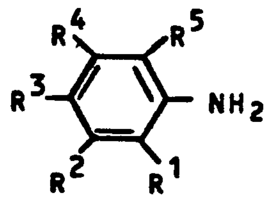

(XI)

in welcher

$R^1$, $R^2$, $R^3$, $R^4$ und $R^5$ die oben angegebene Bedeutung haben,

mit Natriumnitrit, in Gegenwart einer Säure, wie beispielsweise Schwefelsäure, und dann mit Zinn-(II)-chlorid, ebenfalls in Gegenwart einer Säure, wie beispielsweise Salzsäure, bei Temperaturen zwischen -20°C und +80°C umsetzt oder alternativ die bekannten Chlorbenzole der Formel (XIa),

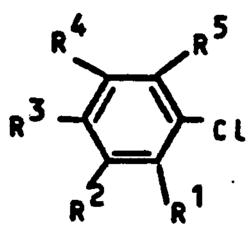

(XIa)

in welcher

$R^1$, $R^2$, $R^3$, $R^4$ und $R^5$ die oben angegebene Bedeutung haben,

mit Hydrazinhydrat gegebenenfalls in Gegenwart eines Verdünnungsmittels wie beispielsweise Dioxan bei Temperaturen zwischen 20°C und 150°C umsetzt.

Die zur Durchführung des erfindungsgemäßen Verfahrens (b) als Ausgangsstoffe benötigten 5-Amino-4-nitropyrazole sind durch die Formel (III) allgemein definiert. In dieser Formel stehen $R^1$, $R^2$, $R^3$, $R^4$ und $R^5$ vorzugsweise für diejenigen Substituenten, die bereits im Zusammenhang mit der Beschreibung der erfindungsgemäßen Stoffe der Formel (I) als bevorzugt für diese Reste genannt wurden.

Die 5-Amino-4-nitropyrazole der Formel (III) sind ebenfalls noch nicht bekannt. Sie sind jedoch Gegenstand einer eigenen nicht vorveröffentlichten Patentanmeldung (DE-P-3 402 308 vom 24.01.1984).

Man erhält sie beispielsweise, wenn man Phenylhydrazine der Formel (VIII),

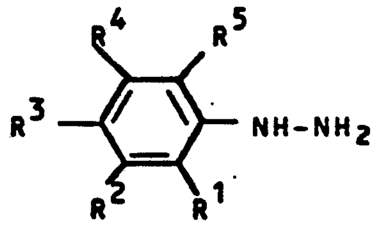

(VIII)

in welcher
R¹, R², R³, R⁴ und R⁵ die oben angegebene Bedeutung haben, entweder mit Acrylnitril-Derivaten der Formel (XII),

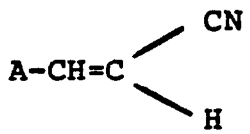

(XII)

in welcher
A für Halogen, Hydroxy oder Alkoxy, insbesondere für Chlor, Brom, Hydroxy, Methoxy oder Ethoxy steht, oder mit 2-Halogenacrylnitrilen der Formel (XIIa),

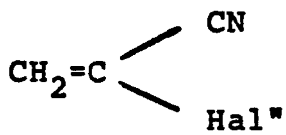

(XIIa)

in welcher
Hal'' für Halogen, insbesondere für Chlor oder Brom steht, oder mit 2,3-Dihalogenpropionitrilen der Formel (XIIb)

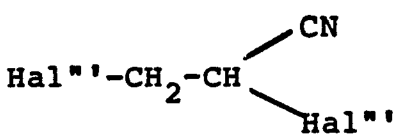

(XIIb)

in welcher
Hal''' für Halogen, insbesondere für Chlor oder Brom steht, zunächst in einer Stufe, gegebenenfalls in Gegenwart eines Verdünnungsmittels wie beispielsweise Methanol, sowie gegebenenfalls in Gegenwart eines Reaktionshilfsmittels, wie beispielsweise Essigsäure, bei Temperaturen zwischen -20° C und +20° C umsetzt zu den Phenylhydrazinen der Formel (XIII),

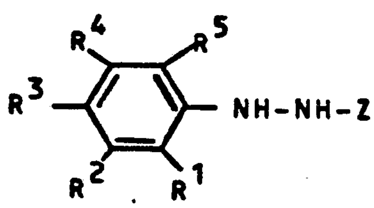

(XIII)

in welcher
R¹, R², R³, R⁴ und R⁵ die oben angegebene Bedeutung haben und
Z für einen der Reste

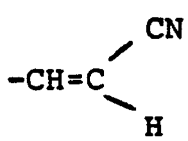 , 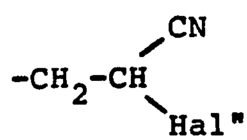

oder

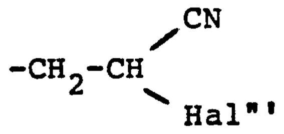

steht, wobei Hal" und Hal" ' die oben angegebene Bedeutung haben,

und für gleiche oder unterschiedliche Halogenatome stehen,

und diese in einer 2. Stufe, gegebenenfalls in Gegenwart eines Verdünnungsmittels, wie beispielsweise Methanol, und gegebenenfalls in Gegenwart eines Säurebindemittels, wie beispielsweise Natriumcarbonat, bei Temperaturen zwischen +50°C und +150°C cyclisiert, oder direkt in einem Reaktionsschritt ohne Isolierung der Zwischenstufe der Formel (XIII), gegebenenfalls in Gegenwart eines Verdünnungsmittels, wie beispielsweise Methanol, bei Temperaturen zwischen +50°C und +150°C cyclisiert zu den 5-Amino-pyrazolen der Formel (XIV),

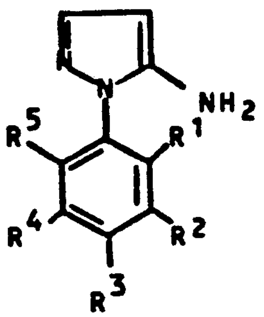

(XIV)

in welcher

$R^1$, $R^2$, $R^3$, $R^4$ und $R^5$ die oben angegebene Bedeutung haben,

und diese in einer 3. Stufe mit Salpetersäure, gegebenenfalls in Gegenwart eines Verdünnungsmittels, wie beispielsweise Essigsäure, und gegebenenfalls in Gegenwart eines Katalysators, wie beispielsweise Acetanhydrid, bei Temperaturen zwischen -20°C und +150°C nitriert.

Die Acrylnitril-Derivate der Formel (XII) sind bekannt (vgl. z. B. DE-OS-3 129 429, EP-34 945; J. Chem. Soc. D 1970, 1255; Can. J. Chem. 48, (1970) 2104-2109, J. Heterocyclic Chem. 19, (1982) 1267-1273; Can. J. Chem. 51, (1973) 1239-1244) oder können nach literaturbekannten Verfahren in einfacher analoger Weise erhalten werden.

Die 2-Halogenacrylnitrile der Formel (XIIa) und die 2,3-Dihalogen-propionitrile der Formel (XIIb) sind ebenfalls bekannt (vgl. z. B. J. Prakt. Chem 321, (1979) 93, J. Heterocyclic Chem. 19, (1982) 1265; J. Heterocyclic Chem. 19, (1982) 1267).

Die zur Durchführung des erfindungsgemäßen Verfahrens (b) weiterhin als Ausgangsstoffe benötigen Nitritverbindungen sind durch die Formel (IV) allgemein definiert. In dieser Formel steht R vorzugsweise für Wasserstoff, für ein Natrium- oder Kaliumkation oder für geradkettiges oder verzweigtes Alkyl mit 1 bis 4 Kohlenstoffatomen.

Die Nitritverbindungen der Formel (IV) sind allgemein bekannte Grundchemikalien.

Die zur Durchführung des erfindungsgemäßen Verfahrens (b) weiterhin als Ausgangsstoffe benötigten Haloforme sind durch die Formel (V) allgemein definiert. In dieser Formel (V) steht Hal vorzugsweise für Chlor, Brom oder Iod.

Die Haloforme der Formel (V) sind ebenfalls allgemein bekannte Verbindungen der organischen Chemie.

Bei der Durchführung des erfindungsgemäßen Verfahrens (c) werden als Ausgangsstoffe die erfindungsgemäßen Verbindungen der 1-Aryl-4-nitropyrazole der Formel (I) eingesetzt, die mit Hilfe der erfindungsgemäßen Verfahren (a) und (b) erhältlich sind.

Die zur Durchführung des erfindungsgemäßen Verfahrens (c) weiterhin als Ausgangsstoffe benötigten Halogenide sind durch die Formel (VI) allgemein definiert. In dieser Formel (VI) steht $M^{\oplus}$ vorzugsweise für ein Natrium- oder Kaliumkation oder für ein Ammonium- oder Tetraalkylammoniumion, wobei als Alkylreste solche mit 1 bis 12 Kohlenstoffatomen in Frage kommen. Hal' steht für Fluor, Chlor, Brom oder Iod.

Die Halogenide der Formel (VI) sind ebenfalls allgemein bekannte Verbindungen der organischen Chemie.

Als Verdünnungsmittel zur Durchführung des erfindungsgemäßen Verfahrens (a) kommen alle üblicherweise für derartige Nitrierungsreaktionen verwendbaren Lösungsmittel in Frage. Vorzugsweise verwendet man die als Reagenzien in Frage kommenden Säuren oder deren Gemische mit Katalysatorsäure, wie beispielsweise Schwefelsäure, Salpetersäure, Acetanhydrid oder Nitriersäure, gleichzeitig als Verdünnungsmittel. Es kommen gegebenenfalls auch inerte organische Lösungsmittel, wie beispielsweise Eisessig oder chlorierte Kohlenwasserstoffe, wie Methylenchlorid, Chloroform oder Tetrachlorkohlenstoff als Verdünnungsmittel in Frage.

Als Katalysatoren oder Reaktionshilfsmittel zur Durchführung des erfindungsgemäßen Verfahrens (a) kommen ebenfalls die für derartige Nitrierungen üblichen Katalysatoren in Frage; vorzugsweise verwendet man saure Katalysatoren, wie beispielsweise Schwefelsäure oder Acetanhydrid.

Die Reaktionstemperaturen können bei der Durchführung des erfindungsgemäßen Verfahrens (a) in einem größeren Bereich variiert werden. Im allgemeinen arbeitet man zwischen -50°C und +200°C, vorzugsweise zwischen -20°C und +150°C.

Zur Durchführung des erfindungsgemäßen Verfahrens (a) setzt man pro Mol 1-Arylpyrazol der Formel (II) im allgemeinen 1,0 bis 100 Mol, vorzugsweise 1,0 bis 50 Mol an Salpetersäure und gegebenenfalls 0,1 bis 10 Mol an Katalysator ein.

Die Reaktionsführung, Aufarbeitung und Isolierung der Reaktionsprodukte der Formel (I) erfolgt in allgemein üblicher Art und Weise.

Als Verdünnungsmittel zur Durchführung des erfindungsgemäßen Verfahrens (b) kommen alle üblicherweise für derartige Diazotierungsreaktionen verwendbaren Lösungsmittel, wie beispielsweise Essigsäure oder Dioxan, in Frage. Vorzugsweise verwendet man einen entsprechenden Überschuß an dem gleichzeitig als Reaktionspartner eingesetzten Haloform als Verdünnungsmittel. Auch ein entsprechender Überschuß an gegebenenfalls verwendbarer Katalysatorsäure läßt sich gleichzeitig als Verdünnungsmittel verwenden.

Als Katalysatoren zur Durchführung des erfindungsgemäßen Verfahrens (b) kommen alle für derartige Diazotierungsreaktionen üblichen Katalysatoren in Frage. Vorzugsweise verwendet man Säuren, wie beispielsweise Salzsäure, Bromwasserstoffsäure oder Schwefelsäure.

Die Reaktionstemperaturen zur Durchführung des erfindungsgemäßen Verfahrens (b) können in gewissen Bereichen variiert werden. Im allgemeinen arbeitet man zwischen -20°C und +120°C, vorzugsweise zwischen 0°C und +80°C.

Zur Durchführung des erfindungsgemäßen Verfahrens (b) setzt man pro Mol 5-Amino-4-nitropyrazol der Formel (III) im allgemeinen 1,0 bis 100 Mol an Haloform der Formel (V) und 1,0 bis 10 Mol an Nitrit der Formel (IV) ein. Die Reaktionsführung, Aufarbeitung und Isolierung der Reaktionsprodukte erfolgt in Analogie zu bekannten Verfahren (vgl. z. B. "Organikum" 15. Auflage, VEB Deutscher Verlag der Wissenschaften Berlin 1981, S. 652 ff oder J. Chem. Soc. C 1966, 1249 oder Rev. Latinoam. Quim. 13, (1982) 100-102.

Als Verdünnungsmittel zur Durchführung des erfindungsgemäßen Verfahrens (c) kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Ketone, wie Aceton oder Butanon, Nitrile, wie Acetonitril oder Propionitril, Amide, wie Dimethylformamid, Dimethylacetamid, N-Methylformanilid, N-Methylpyrrolidon oder Hexamethylphosphorsäuretriamid, Ester, wie Essigsäureethylester, Sulfoxide oder Sulfone, wie Dimethylsulfoxid oder Sulfolan oder Alkohole, wie Methanol, Ethanol oder Propanol.

Als Katalysatoren zur Durchführung des erfindungsgemäßen Verfahrens (c) kommen quartäre Ammonium- oder Phosphoniumsalze oder cyclische Polyether in Frage.

Als Beispiele für solche Katalysatoren seien genannt:

Tetrabutylammoniumiodid, Tetrabutylammoniumbromid, Tributylmethylphosphoniumbromid, Trimethyl-$C_{13}/C_{15}$-alkyl-ammoniumchlorid, Dibenzyl-dimethyl-ammonium-methylsulfat, Dimethyl-$C_{12}/C_{14}$-alkyl-benzylammoniumchlorid, Tetrabutylammoniumhydroxid, 18-Krone-6, Triethylbenzylammoniumchlorid, Trimethylbenzylammoniumchlorid.

Die Reaktionstemperatur kann bei der Durchführung des erfindungsgemäßen Verfahrens (c) innerhalb eines größeren Bereichs variiert werden. Sie liegt im allgemeinen zwischen 0°C und +120°C, vorzugsweise zwischen +20°C und +80°C.

Zur Durchführung des erfindungsgemäßen Verfahrens (c) setzt man pro Mol an Ausgangsverbindung der Formel (I) im allgemeinen 1,0 bis 50 Mol, vorzugsweise 1,0 bis 20 Mol an Halogenid der Formel (VI) und 0,01 bis 1,0 Mol an Phasentransferkatalysator ein. Die Reaktionsdurchführung, Aufarbeitung und Isolierung der Reaktionsprodukte erfolgt in Analogie zu bekannten Verfahren (vgl. z. B. "Organikum", 15. Auflage, VEB Deutscher Verlag der Wissenschaften Berlin 1981, S. 421 ff).

Die erfindungsgemäßen Verfahren (a), (b) und (c) werden im allgemeinen unter Normaldruck durchgeführt. Es ist jedoch auch möglich, unter erhöhtem oder vermindertem Druck zu arbeiten.

Die erfindungsgemäßen Verbindungen der Formel (I) können neben ihrer Verwendung als herbizide Wirkstoffe auch als Zwischenprodukte zur Synthese weiterer Wirkstoffe verwendet werden. Sie lassen sich beispielsweise mit Aminen umsetzen zu den entsprechenden ebenfalls herbizid wirksamen 5-Amino-1-aryl-4-nitropyrazolen, welche Gegenstand einer eigenen vorgängigen Patentanmeldung sind (vgl. DE-P-3 402 308 vom 24.01.1984).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z. B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z. B. auf Industrie- und Gleisanlagen und auf Wegen und Plätzen mit und ohne Baumbewuchs. Ebenso können die Verbindungen zur Unkrautbekämpfung in Dauerkulturen, z. B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei zeigen die erfindungsgemäßen Wirkstoffe der Formel (I) neben einer besonders guten allgemeinherbiziden Wirksamkeit auch eine deutlich verbesserte Kulturpflanzenselektivität in wichtigen Kulturen und können als selektive Unkrautbekämpfungsmittel in dikotylen Kulturen, wie beispielsweise Zuckerrüben, Baumwollpflanzungen, Sojabohnen oder Erdnüssen, als auch in monokotylen Kulturen, insbesondere Getreide, wie beispielsweise Weizen, eingesetzt werden.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoffimprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z. B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z. B. Erdölfraktionen, mineralische und pflanzliche Öle, Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate,

als feste Trägerstoffe für Granulate kommen in Frage:

z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln;

als Emulgier- und/oder schaumerzeugende Mittel kommen in Frage:

z. B. nichtionogene und anionische Emulgatoren, wie Polyoxyethylen-Fettsäure-Ester, Polyoxyethylen-Fettalkohol-Ether, z. B. Alkylaryl-polyglykolether, Alkylsulfonate, Alkylsulfate, Arylsulfonate sowie Eiweißhydrolysate;

als Dispergiermittel kommen in Frage:

z. B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z. B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z. B.
1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide;
4-Amino-3-methyl-6-phenyl-1,2,4-triazin-5(4H)-on zur Unkrautbekämpfung in Zuckerrüben und 4-Amino-6-(1,1-dimethylethyl)-3-methylthio-1,2,4-triazin-5(4H)-on zur Unkrautbekämpfung in Sojabohnen, in Frage.
Auch Mischungen mit Heteroaryl-oxyacetamiden;
N,N-Dimethyl-N'-(3-chlor-4-methylphenyl)-harnstoff,
N,N-Dimethyl-N'-(4-isopropylphenyl)-harnstoff,
4-Amino-6-t-butyl-3-ethylthio-1,2,4-triazin-5(4H)-on,
2,4-Dichlorphenoxyessigsäure,
2,4-Dichlorphenoxypropionsäure,
(2-Methyl-4-chlorphenoxy)-essigsäure,
(4-Chlor-2-methylphenoxy)-propionsäure,
Chloressigsäure-N-(methoxymethyl)-2,6-diethylanilid,
O-(Methoxycarbonylaminophenyl)-N-phenyl-carbamat;
2,6-Dinitro-4-trifluormethyl-N,N-dipropylanilin,
2-Ethoxy-2,3-dihydro-3,3-dimethyl-5-benzofuranyl-methansulfonat;
Butyl-2-<4-[(5-trifluormethyl-2-pyridinyl)-oxy]-phenoxy >-propanoat;
N,N-Diisopropyl-S-(2,3,3-trichlorallyl)-thiocarbamat;
5-Amino-4-chlor-2-phenyl-2,3-dihydro-3-oxo-pyridazin;
Methyl-6,6-dimethyl-2,4-dioxo-3-[1-(2-propenyloxyamino)-butyliden]-cyclohexancarbonsäure;
3,5-Diiod-4-hydroxybenzonitril;
3-Isopropyl-2,1,3-benzothiadiazin-4-on-2,2-dioxid;
2-Chlor-N-[(4-methoxy-6-methyl-1,3,5-triazin-2-yl)-aminocarbonyl]-benzolsulfonamid;
4-Ethylamino-2-t-butylamino-6-methylthio-s-triazin;
N-(1-Ethylpropyl)-3,4-dimethyl-2,6-dinitroanilin und 2-<4-[(3-Chlor-5-trifluormethyl-2-pyridinyl)-oxy]-phenoxy>propansäure bzw. -propansäureethylester sind möglich.

Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z. B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,01 und 5 kg pro ha.

Die Herstellung und die Verwendung der erfindungsgemäßen Wirkstoffe geht aus den nachfolgenden Beispielen hervor.

**Herstellungsbeispiele**

**Beispiel 1** / nach Verfahren (a)

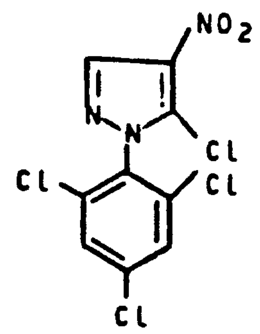

Zu einer Suspension von 7,0 g (0,025 Mol) 5-Chlor-1-(2,4,6-trichlorphenyl)-pyrazol in 30 ml Acetanhydrid tropft man unter Rühren bei Raumtemperatur innerhalb von 2,5 Stunden 26 ml (0,62 Mol) rauchende Salpetersäure so zu, daß die Innentemperatur 35°C nicht übersteigt (ca. 10 ml/Stunde). Nach beendeter Zugabe rührt man 1 Stunde bei Raumtemperatur nach, gießt die Mischung auf Eiswasser, nimmt das ölige Produkt in Chloroform auf, wäscht je zweimal mit gesättigter Natriumcarbonatlösung und Wasser, trocknet über Natriumsulfat und entfernt das Lösungsmittel im Vakuum. Der kristalline Rückstand wird mit Petrolether digeriert, abgesaugt und getrocknet.

Man erhält 6,0 g (73 % der Theorie) an 5-Chlor-4-nitro-1-(2,4,6-trichlorphenyl)-pyrazol vom Schmelzpunkt 78°C - 84°C.

**Beispiel 2** / nach Verfahren (b)

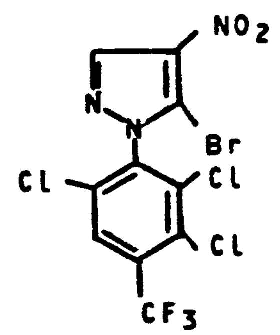

5,2 g (0,014 Mol) 5-Amino-4-nitro-1-(2,3,6-trichlor-4-trifluormethylphenyl)-pyrazol in 16 ml (0,18 Mol) Bromoform werden tropfenweise unter Rühren innerhalb von 10 Minuten mit 4,8 ml (0,04 Mol) t-Butyl-nitrit versetzt, wobei die Temperatur der Reaktionsmischung auf 50°C ansteigt. Nach beendeter Zugabe rührt man eine weitere Stunde bei Rückflußtemperatur, engt die Mischung im Vakuum ein und reinigt das zurückbleibende Öl säulenchromatographisch (Kieselgel/Laufmittel: Chloroform/Aceton 9 : 1).

Man erhält 5,0 g (82 % der Theorie) an 5-Brom-4-nitro-1-(2,3,6-trichlor-4-trifluormethylphenyl)-pyrazol vom Schmelzpunkt 97°C - 99°C.

In entsprechender Weise und gemäß den allgemeinen Herstellungsangaben erhält man die in der folgenden Tabelle aufgeführten 1-Aryl-4-nitropyrazole der allgemeinen Formel (I):

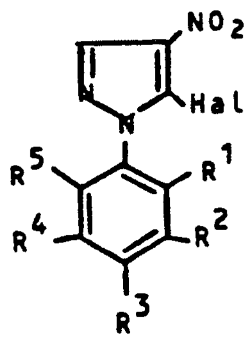

(I)

**Tabelle 2**

| Bsp. Nr. | Hal | $R^1$ | $R^2$ | $R^3$ | $R^4$ | $R^5$ | Schmelzpunkt (°C) |
|---|---|---|---|---|---|---|---|
| 3 | Cl | Cl | H | $CF_3$ | H | Cl | 82 - 84 |
| 4 | Cl | Cl | H | $OCF_3$ | H | H | 64 - 70 |
| 5 | Br | Cl | H | $CF_3$ | H | Cl | 96 |
| 6 | Br | Cl | H | $CF_3$ | H | Br | 89 - 97 |
| 7 | Br | $OCF_3$ | H | Br | H | Br | 86 |
| 8 | Br | Cl | H | $SO_2CF_3$ | H | Cl | 142 - 144 |
| 9 | Br | Cl | H | $CF_3$ | H | H | Öl |
| 10 | Br | Cl | Cl | $SCF_3$ | H | Cl | Öl |
| 11 | Br | Cl | H | $OCF_3$ | H | H | 83 |
| 12 | Br | Cl | Cl | $OCF_3$ | H | Cl | Öl |

**Herstellung der Ausgangsverbindungen**

**Beispiel II-1**

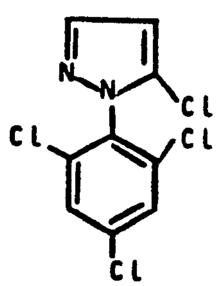

200 g (0,76 Mol) 1-(2,4,6-Trichlorphenyl)-4H-$\Delta^2$-pyrazolin-5-on werden zusammen mit 500 ml (837,5 g = 2,95 Mol) Phosphoroxychlorid 16 Stunden lang im Autoklaven auf 160°C erhitzt. Zur Aufarbeitung gießt man den erkalteten Reaktionsansatz auf Eis, rührt 15 Minuten, saugt den Niederschlag ab, wäscht mit Wasser nach, verrührt zur Reinigung den Rückstand 0,5 Stunden bei ca. 40°C in konzentrierter wäßriger Ammoniaklösung, saugt abermals ab, wäscht mit Wasser nach und trocknet. Zur weiteren Reinigung löst man das trockene Pulver in Chloroform, wäscht mit Wasser, trocknet die Chloroformphase über Natriumsulfat und entfernt das Lösungsmittel im Vakuum.

Man erhält 118 g (55 % der Theorie) an 5-Chlor-1-(2,4,6-trichlorphenyl)-pyrazol vom Schmelzpunkt 96°C.

In entsprechender Weise und gemäß den allgemeinen Angaben zur Herstellung erhält man die in der folgenden Tabelle aufgeführten 1-Aryl-5-halogenpyrazole der allgemeinen Formel (II):

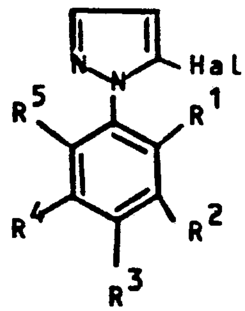

(II)

**Tabelle 3**

**Beispiel II-2**

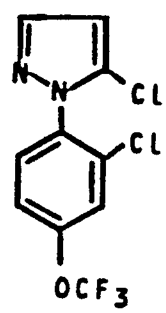

Oel

**Beispiel II-3**

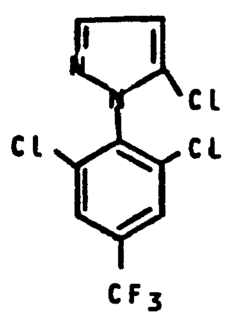

Schmelzpunkt 70 - 72° C

**Beispiel III-1**

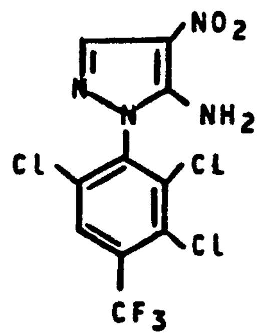

7,5 g (0,0227 Mol) 5-Amino-1-(2,3,6-trichlor-4-trifluormethylphenyl)-pyrazol (vgl. Bsp. XIV-1) und 2,2 ml (2,3 g = 0,023 Mol) Acetanhydrid in 20 ml Eisessig werden ca. 4 Stunden bei Raumtemperatur gerührt, bis im Dünnschichtchromatogramm kein Ausgangsprodukt mehr nachweisbar ist. Dann gibt man weitere 2,7 ml (2,9 g = 0,0288 Mol) Acetanhydrid und bei 5°C bis 10°C 1,2 ml (1,8 g = 0,028 Mol) 98-prozentige Salpetersäure zu, rührt 8 Stunden bei Raumtemperatur, entfernt das Lösungsmittel im Vakuum und nimmt den Rückstand in 30 ml Ethanol und 20 ml konzentrierter Salzsäure auf.

Man erhitzt für 12 Stunden auf Rückflußtemperatur, engt im Vakuum ein, nimmt den Rückstand in 100 ml Dichlormethan auf, wäscht vorsichtig ($CO_2$-Entwicklung) mit 100 ml gesättigter Natriumhydrogencarbonatlösung, trocknet über Magnesiumsulfat und entfernt das Lösungsmittel im Vakuum.

Man erhält 7,5 g (84 % der Theorie) an 5-Amino-4-nitro-1-(2,3,6-trichlor-4-trifluormethylphenyl)-pyrazol vom Schmelzpunkt 78°C bis 85°C.

**Beispiel VIII-1**

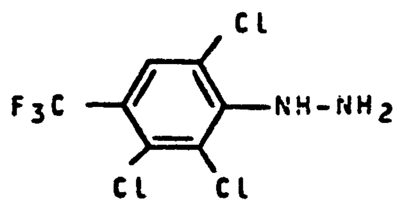

200 g (0,704 Mol) 1,2,3,4-Tetrachlor-5-trifluormethylbenzol und 240 ml (247,2 g/4,94 Mol) Hydrazinhydrat in 500 ml Dioxan werden 14 Stunden lang unter Rückfluß erhitzt. Von der abgekühlten zweiphasigen Reaktionsmischung trennt man die schwerere (wäßrige) Phase ab und engt die organische Phase im Vakuum zur Trockne ein. Der Rückstand wird in 600 ml Wasser und 100 ml Dichlormethan suspendiert, mit 10-prozentiger wäßriger Natriumhydroxidlösung auf pH 10 eingestellt und langsam auf 30°C bis 35°C erwärmt, wobei sich aus der trüben Suspension zwei klare Phasen bilden. Man läßt auf Raumtemperatur abkühlen, trennt die organische Phase ab, wäscht sie mit 200 ml konzentrierter wäßriger Natriumchloridlösung, trocknet über Magnesiumsulfat und entfernt das Lösungsmittel im Vakuum. Das Rohprodukt wird in siedendem Hexan 3 bis 4 Stunden gerührt, danach 15 Stunden bei 0°C bis 5°C gekühlt, kalt abgesaugt und 2 bis 3 Stunden bei 50°C im Vakuum getrocknet.

Man erhält 146 g (71,2 % der Theorie) an 2,3,6-Trichlor-4-trifluormethyl-phenylhydrazin vom Schmelzpunkt 67 - 70°C mit einem gaschromatographisch bestimmten Gehalt von 96 %.

In entsprechender Weise oder gemäß den allgemeinen Angaben zur Herstellung erhält man

**Beispiel VIII-2**

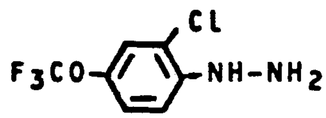

Schmelzpunkt 35° C

**Beispiel VIII-3**

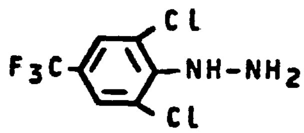

Schmelzpunkt 56 - 57° C

**Beispiel VIIIa-1**

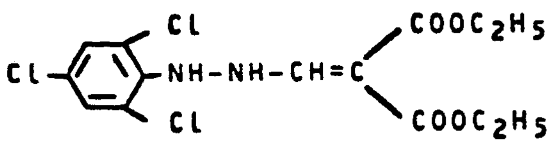

105 g (0,5 Mol) 2,4,6-Trichlorphenylhydrazin und 108 g (0,5 Mol) Ethoxymethylenmalonsäurediethylester werden in 500 ml Ethanol 5 Stunden unter Rückfluß erhitzt, anschließend 12 Stunden bei Raumtemperatur gerührt, dann abgekühlt auf -10° C und der Rückstand abfiltriert und getrocknet.

Man erhält N-[2,2-Bis-(ethoxycarbonyl)-vinyl]-N'-(2,4,6-trichlorphenyl)-hydrazin vom Schmelzpunkt 92° C.

In entsprechender Weise erhält man

**Beispiel VIIIa-2**

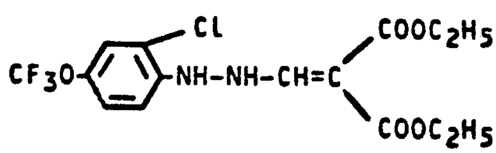

Schmelzpunkt 56° - 57° C

**Beispiel VIIIa-3**

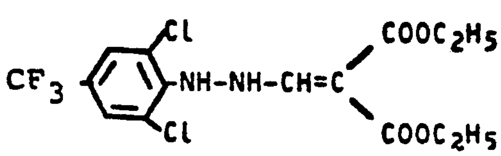

Schmelzpunkt 77° - 83° C

**Beispiel IX-1**

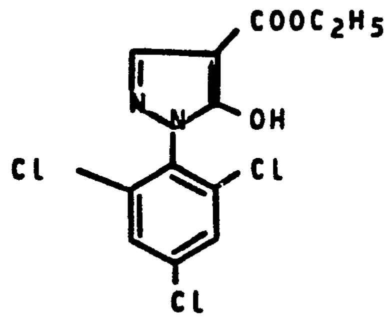

35,8 g (0,094 Mol) N-[2,2-Bis(ethoxycarbonyl)-vinyl]-N'-(2,4,6-trichlorphenyl)-hydrazin werden langsam auf 170°C erhitzt und für 45 Minuten bei 170°C bis 175°C gerührt, wobei gleichzeitig 11 ml Ethanol abdestilliert werden und der Rückstand langsam fest wird.

Der erhaltene Rückstand wird mit Acetonitril verrührt und abgesaugt.

Man erhält 26 g (82,4 % der Theorie) an 4-Ethoxycarbonyl-5-hydroxy-1-(2,4,6-trichlorphenyl)-pyrazol vom Schmelzpunkt 228°C.

In entsprechender Weise erhält man

**Beispiel IX-2**

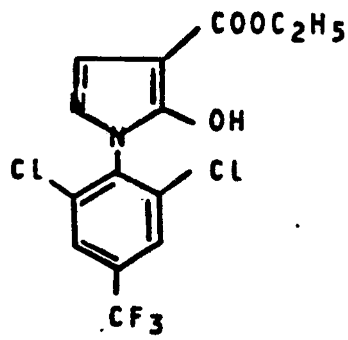

Schmelzpunkt 188°C

**Beispiel X-1**

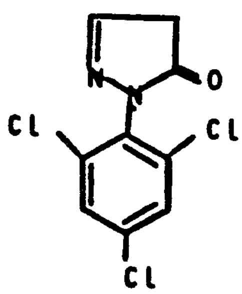

5 g (0,015 Mol) 4-Ethoxycarbonyl-5-hydroxy-1-(2,4,6-trichlorphenyl)-pyrazol in 75 ml Ethanol werden mit 3 g (0,075 Mol) Natriumhydroxid - in 3 ml Wasser gelöst - versetzt und 12 Stunden unter Rückfluß erhitzt; man läßt die Mischung abkühlen, setzt weitere 3 g (0,075 Mol) Natriumhydroxid, in 3 ml Wasser gelöst, zu und erhitzt für abermals 12 Stunden auf Rückflußtemperatur. Wenn im Dünnschichtchromatogramm kein Ausgangsprodukt mehr nachweisbar ist, säuert man mit konzentrierter Salzsäure an, erhitzt wiederum für 3 Stunden auf Rückflußtemperatur, filtriert die erhaltene Reaktionsmischung, engt das Filtrat ein, nimmt den Rückstand in Chloroform auf, wäscht mit Wasser, trocknet über Natriumsulfat und entfernt das Lösungsmittel im Vakuum.

Man erhält 1 g (25,3 % der Theorie) an 1-(2,4,6-Trichlorphenyl)-4H-$\Delta^2$-pyrazolin-5-on vom Schmelzpunkt 202°C.

(Alternative Herstellungsmöglichkeit):

100 g (0,26 Mol) N-[2,2-Bis(ethoxycarbonyl)-vinyl]-N'-(2,4,6-trichlorphenyl)-hydrazin und 200 g (5 Mol) Natriumhydroxid werden in 1400 ml Wasser 12 Stunden refluxiert. Die erkaltete Reaktionsmischung wird unter heftigem Rühren mit konzentrierter Salzsäure vorsichtig angesäuert ($CO_2$-Entwicklung), der ausgefallene Feststoff abgesaugt und an der Luft getrocknet. Er läßt sich aus Ethanol umkristallisieren.

Man erhält 53 g (77 % der Theorie) an 1-(2,4,6-trichlorphenyl)-4H-$\Delta^2$-pyrazolin-5-on vom Schmelzpunkt 202°C.

In entsprechender Weise und gemäß den allgemeinen Angaben zur Herstellung erhält man die folgenden Pyrazolinone der Formel (X)

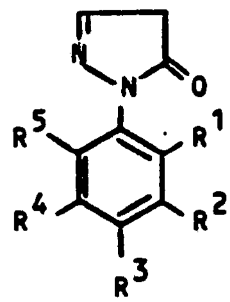

(X)

**Beispiel X-2**

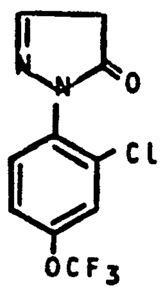

Schmelzpunkt 210°C (Zers.)

**Beispiel X-3**

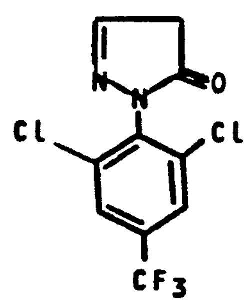

Schmelzpunkt 228°C - 230°C

**Beispiel XIV-1**

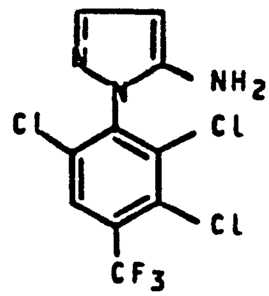

45 g (0,17 Mol) 2,3,6-Trichlor-4-trifluormethyl-phenylhydrazin und 35 mg (0,1 mMol) Ethylendiamintetraessigsäure-Dinatriumsalz (Titriplex III) in 260 ml Methanol werden bei Rückflußtemperatur tropfenweise innerhalb von 30 Minuten mit 57 ml (62,7 g = 0,71 Mol) 2-Chloracrylnitril versetzt und weitere 5 Stunden bei Rückflußtemperatur (ca. 65°C) gerührt. Die Reaktionsmischung wird im Vakuum eingeengt, der Rückstand in 260 ml Methanol aufgenommen und bei Raumtemperatur tropfenweise unter Rühren innerhalb von 15 Minuten mit 15,4 ml (0,541 Mol) konzentrierter Schwefelsäure versetzt. Dabei steigt die Temperatur der Reaktionsmischung auf 32°C. Nach beendeter Zugabe rührt man 30 Stunden bei 55°C, kühlt auf Raumtemperatur ab, setzt 56 g (0,534 Mol) Natriumcarbonat zu, rührt abermals 4 Stunden bei Raumtemperatur, engt im Vakuum ein, nimmt den Rückstand in 550 ml Dichlormethan auf, gibt 55 ml Wasser zu, rührt 8 Stunden bei Raumtemperatur, trennt die organische Phase ab, wäscht mit 250 ml konzentrierter wäßriger Natriumchloridlösung, trocknet über Magnesiumsulfat und entfernt das Lösungsmittel im Vakuum.

Man erhält 52,6 g (94 % der Theorie) an 5-Amino-1-(2,3,6-trichlor-4-trifluormethyl-phenyl)-pyrazol vom Schmelzpunkt 109°C - 114°C.

**Herstellung von Folgeprodukten**

**Beispiel (III-2)**

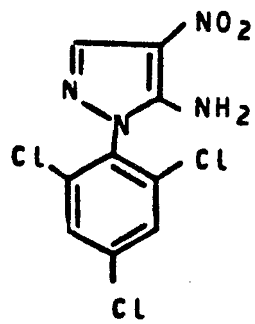

2,8 g (0,0086 Mol) 5-Chlor-4-nitro-1-(2,4,6-trichlorphenyl)-pyrazol werden in 100 ml mit Ammoniak gesättigtem Ethanol 10 Stunden im Bombenrohr oder Autoklav auf 160°C erhitzt. Die erkaltete Reaktionsmischung wird eingeengt, in Chloroform aufgenommen, mit Wasser gewaschen, über Natriumsulfat getrocknet und im Vakuum eingeengt. Der kristalline Rückstand wird aus Toluol umkristallisiert.

Man erhält 2,2 g (85 % der Theorie) an 5-Amino-4-nitro-1-(2,4,6-trichlorphenyl)-pyrazol vom Schmelzpunkt 218°C - 220°C.

**Anwendungsbeispiele:**

In den folgenden Anwendungsbeispielen wurde die nachstehend aufgeführte Verbindung als Vergleichssubstanz eingesetzt:

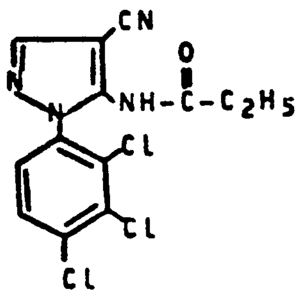

(A)

4-Cyano-5-propionamido-1-(2,3,4-trichlor-phenyl)-pyrazol (bekannt aus DE-OS-3 226 513).

**Beispiel A**

**Pre-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z. B. die Verbindung gemäß dem Herstellungsbeispiel: (3)

**Beispiel B**

**Post-emergence-Test**

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.

Es bedeuten:

0 % = keine Wirkung (wie unbehandelte Kontrolle)
100 % = totale Vernichtung

Eine deutliche Überlegenheit in der Nutzpflanzenselektivität gegenüber dem Stand der Technik zeigt in diesem Test z. B. die Verbindung gemäß dem Herstellungsbeispiel: (3).

**Patentansprüche**

1. 1-Aryl-4-nitropyrazole der allgemeinen Formel (I),

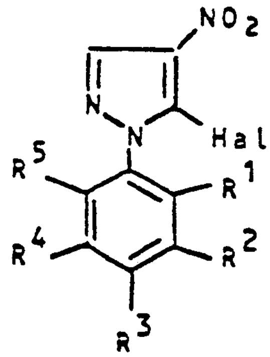

(I)

in welcher

Hal für Halogen steht

$R^1$ und $R^3$ unabhängig voneinander für Cyano, Nitro, Halogen oder für jeweils geradkettiges oder verzweigtes Alkyl, Alkoxy oder Alkoxycarbonyl mit jeweils 1 bis 4 Kohlenstoffatomen im Alkylteil, für jeweils geradkettiges oder verzweigtes Halogenalkyl oder Halogenalkoxy mit jeweils 1 bis 4 Kohlenstoffatomen und 1 bis 9 gleichen oder verschiedenen Halogenatomen oder für einen Rest $-S(O)_n-R^6$ stehen, und

$R^2$, $R^4$ und $R^5$ unabhängig voneinander und unabhängig von $R^1$ und $R^3$ für die gleichen Reste wie $R^1$ und $R^3$ und zusätzlich für Wasserstoff stehen,

wobei

$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

n für eine Zahl 0, 1 oder 2 steht.

2. 1-Aryl-4-nitropyrazole der Formel (I) gemäß Anspruch 1 in welcher

Hal für Fluor, Chlor, Brom oder Iod steht,

$R^1$ und $R^3$ unabhängig voneinander für Cyano, Nitro, Fluor, Chlor, Brom, Iod, Methyl, Ethyl, n- und i-Propyl, n-, i-, s- und t-Butyl, für Methoxy, Ethoxy, Methoxycarbonyl, Ethoxycarbonyl, für Trifluormethyl, Trichlormethyl, Dichlorfluormethyl, Difluorchlormethyl, Chlormethyl, Dichlormethyl, Difluormethyl, Pentafluorethyl, Tetrafluorethyl, Trifluorchlorethyl, Trifluorethyl, Difluordichlorethyl, Trifluordichlorethyl, Pentachlorethyl, für Trifluormethoxy, Trichlormethoxy, Dichlorfluormethoxy, Difluorchlormethoxy, Chlormethoxy, Dichlormethoxy, Difluormethoxy, Pentafluorethoxy, Tetrafluorethoxy, Trifluorchlorethoxy, Trifluorethoxy, Difluordichlorethoxy, Trifluordichlorethoxy, Pentachlorethoxy oder für einen Rest $-S(O)_n-R^6$ stehen und

$R^2$, $R^4$ und $R^5$ unabhängig voneinander und unabhängig von $R^1$ und $R^3$ für die gleichen Reste wie $R^1$ und $R^3$ und zusätzlich für Wasserstoff stehen, wobei

$R^6$ für Amino, Methylamino, Ethylamino, Dimethylamino, Diethylamino, Fluordichlormethyl, Difluormethyl, Tetrafluorethyl, Trichlorethyl, Trifluormethyl, Methyl oder Ethyl steht

und

n für eine Zahl 0, 1 oder 2 steht.

3. Verfahren zur Herstellung von 1-Aryl-4-nitropyrazolen der Formel (I)

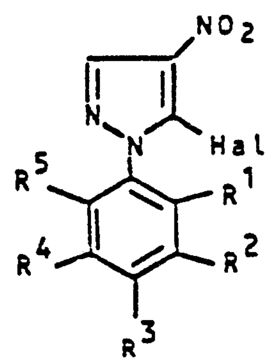

(I)

in welcher

Hal für Halogen steht

$R^1$ und $R^3$ unabhängig voneinander für Cyano, Nitro, Halogen oder für jeweils geradkettiges oder verzweigtes Alkyl, Alkoxy oder Alkoxycarbonyl mit jeweils 1 bis 4 Kohlenstoffatomen im Alkylteil, für jeweils geradkettiges oder verzweigtes Halogenalkyl oder Halogenalkoxy mit jeweils 1 bis 4 Kohlenstoffatomen und 1

bis 9 gleichen oder verschiedenen Halogenatomen oder für einen Rest $-S(O)_2-R^6$ stehen, und

$R^2$, $R^4$ und $R^5$ unabhängig voneinander und unabhängig von $R^1$ und $R^3$ für die gleichen Reste wie $R^1$ und $R^3$ und zusätzlich für Wasserstoff stehen, wobei

$R^6$ für Amino, für jeweils geradkettiges oder verzweigtes Alkyl, Alkylamino, Dialkylamino oder Halogenalkyl mit jeweils 1 bis 4 Kohlenstoffatomen in den einzelnen Alkylteilen und im Fall des Halogenalkyl mit 1 bis 9 gleichen oder verschiedenen Halogenatomen steht und

n für eine Zahl 0, 1 oder 2 steht,

dadurch gekennzeichnet, daß man

(a) 1-Arylpyrazole der allgemeinen Formel (II),

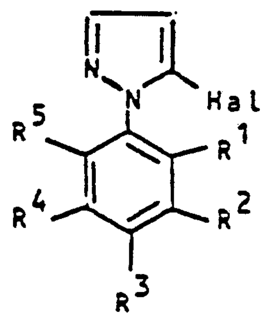

(II)

in welcher

$R^1$, $R^2$, $R^3$, $R^4$, $R^5$ und Hal die oben angegebene Bedeutung haben,

mit Salpetersäure gegebenenfalls in Gegenwart eines Verdünnungsmittels, sowie gegebenenfalls in Gegenwart eines Katalysators umsetzt,

oder

(b) 5-Amino-4-nitropyrazole der Formel (III),

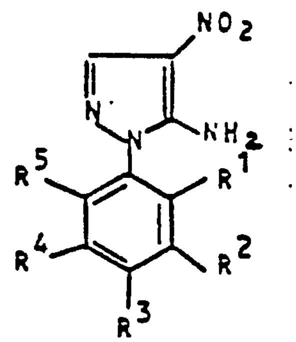

(III)

in welcher

$R^1$, $R^2$, $R^3$, $R^4$, $R^5$ und Hal die oben angegebene Bedeutung haben,

mit Nitritverbindungen der Formel (IV),

$$R\text{-}O\text{-}N=O \qquad \text{(IV)}$$

in welcher

R für Wasserstoff, ein Alkalimetallkation oder für Alkyl steht,

und Haloform der Formel (V),

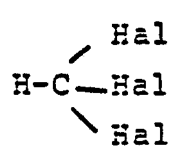

(V)

in welcher

Hal die oben angegebene Bedeutung hat,

gegebenenfalls in Gegenwart eines Katalysators umsetzt, oder daß man

(c) die nach Verfahren (a) oder (b) erhältlichen 1-Aryl-4-nitropyrazole der Formel (I),

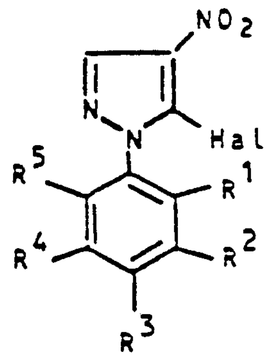

(I)

in welcher $R^1$, $R^2$, $R^3$, $R^4$, $R^5$ und Hal die oben angegebene Bedeutung haben,
mit Halogeniden der Formel (VI),

$M^{\ominus}$ $Hal'^{\ominus}$ (VI)

in welcher
$M^{\ominus}$ für ein Äquivalent eines Metallkations oder für ein gegebenenfalls substituiertes Ammoniumion steht und
$Hal'^{\ominus}$ für Halogen steht, aber verschieden von Hal ist,
gegebenenfalls in Gegenwart eines Verdünnungsmittels und gegebenenfalls in Gegenwart eines Phasentransfer-Katalysators umsetzt.

4. Herbizide Mittel, gekennzeichnet durch einen Gehalt an mindestens einem 1-Aryl-4-nitropyrazol der Formel (I) gemäß den Ansprüchen 1 bis 3.

5. Verfahren zur Bekämpfung von Unkräutern, dadurch gekennzeichnet, daß man 1-Aryl-4-nitropyrazole der Formel (I) gemäß den Ansprüchen 1 bis 3 auf die Unkräuter und/oder ihren Lebensraum ausbringt.

6. Verwendung von 1-Aryl-4-nitropyrazolen der Formel (I) gemäß den Ansprüchen 1 bis 3 zur Bekämpfung von Unkräutern.

7. Verfahren zur Herstellung von herbiziden Mitteln, dadurch gekennzeichnet, daß man 1-Aryl-4-nitropyrazole der Formel (I) gemäß den Ansprüchen 1 bis 3 mit Streckmitteln und/oder oberflächenaktiven Stoffen vermischt.

8. 1-Arylpyrazole der Formel (II)

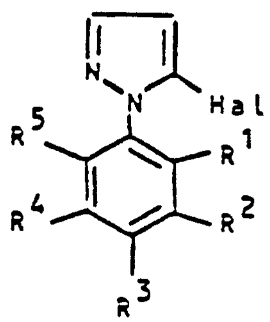

(II)

in welcher
Hal, $R^1$, $R^2$, $R^3$, $R^4$ und $R^5$ die in Anspruch 1 angegebenen Bedeutungen haben.

9. Verfahren zur Herstellung von 1-Arylpyrazolen der Formel (II)

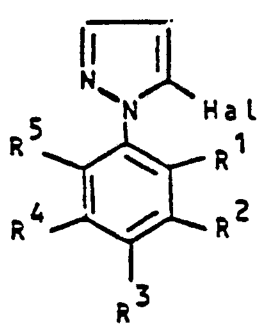

(II)

in welcher
Hal, $R^1$, $R^2$, $R^3$, $R^4$ und $R^5$ die in Anspruch 3 angegebenen Bedeutungen haben,
dadurch gekennzeichnet, daß man entweder Alkoxymethylenmalonester der Formel (VII),

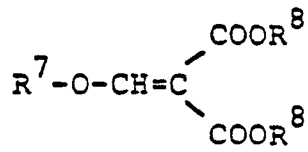

(VII)

in welcher

$R^7$ und $R^8$ unabhängig voneinander jeweils für Alkyl, insbesondere für Methyl oder Ethyl stehen, mit Phenylhydrazinen der Formel (VIII),

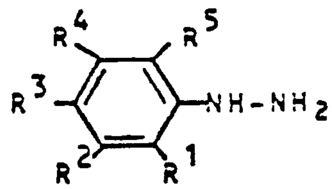

(VIII)

in welcher

$R^1$, $R^2$, $R^3$, $R^4$ und $R^5$ die oben angegebene Bedeutung haben,

zunächst in einer ersten Stufe gegebenenfalls in Gegenwart eines Verdünnungsmittels bei Temperaturen zwischen +10°C und +80°C umsetzt, und die so erhältlichen Pyrazolcarbonsäureester der Formel (IX),

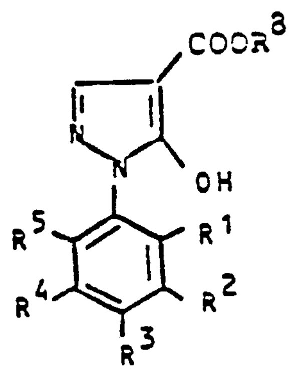

(IX)

in welcher

$R^1$, $R^2$, $R^3$, $R^4$, $R^5$ und $R^8$ die oben angegebene Bedeutung haben

in einer 2. Stufe gegebenenfalls in Gegenwart eines Verdünnungsmittels und gegebenenfalls in Gegenwart einer Base bei Temperaturen zwischen 30°C und 70°C decarboxyliert dann in einer 3. Stufe die so erhältlichen Pyrazolinone der Formel (X),

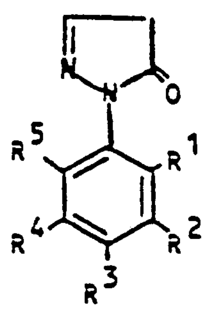

(X)

in welcher

$R^1$, $R^2$, $R^3$, $R^4$ und $R^5$ die oben angegebene Bedeutung haben,

mit Halogenierungsmitteln umsetzt.

## Claims

1. 1-Aryl-4-nitropyrazoles of the general formula (I)

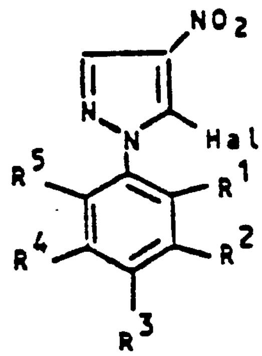

(I)

in which

Hal represents halogen,

$R^1$ and $R^3$ independently of one another represent cyano, nitro or halogen, or represent in each case straight-chain or branched alkyl, alkoxy or alkoxycarbonyl with in each case 1 to 4 carbon atoms in the alkyl part, or represent in each case straight-chain or branched halogenoalkyl or halogenoalkoxy with in each case 1 to 4 carbon atoms and 1 to 9 identical or different halogen atoms, or represent a radical $-S(O)_n-R^6$ and

$R^2$, $R^4$ and $R^5$ independently of one another and independently of $R^1$ and $R^3$ represent the same radicals as $R^1$ and $R^3$ and additionally represent hydrogen,

wherein

$R^6$ represents amino, or in each case represents straight-chain or branched alkyl, alkylamino, dialkylamino or halogenoalkyl with in each case 1 to 4 carbon atoms in the individual alkyl parts and, in the case of halogenoalkyl, with 1 to 9 identical or different halogen atoms and

n represents the number 0, 1 or 2.

2. 1-Aryl-4-nitropyrazoles of the formula (I) according to Claim 1,

in which

Hal represents fluorine, chlorine, bromine or iodine,

$R^1$ and $R^3$ independently of one another represent cyano, nitro, fluorine, chlorine, bromine, iodine, methyl, n- or i-propyl or n-, i-, s- or t-butyl, or represent methoxy, ethoxy, methoxycarbonyl or ethoxycarbonyl, or represent trifluoromethyl, trichloromethyl, dichlorofluoromethyl, difluorochloromethyl, chloromethyl, dichloromethyl, difluoromethyl, pentafluoroethyl, tetrafluoroethyl, trifluorochloroethyl, trifluoroethyl, difluorodichloroethyl, trifluorodichloroethyl or pentachloroethyl, or represent trifluoromethoxy, trichloromethoxy, dichlorofluoromethoxy, difluorochloromethoxy, chloromethoxy, dichloromethoxy, difluoromethoxy, pentafluoroethoxy, tetrafluoroethoxy, trifluorochloroethoxy, trifluoroethoxy, difluorodichloroethoxy, trifluorodichloroethoxy or pentachloroethoxy, or represent a radical $-S(O)_n-R^6$ and

$R^2$, $R^4$ and $R^5$ independently of one another and independently of $R^1$ and $R^3$ represent the same radicals as $R^1$ and $R^3$ and additionally represent hydrogen,

wherein

$R^6$ represents amino, methylamino, ethylamino, dimethylamino, diethylamino, fluorodichloromethyl, difluoromethyl, tetrafluoroethyl, trichloroethyl, trifluoromethyl, methyl or ethyl and

n represents the number 0, 1 or 2.

3. Process for the preparation of 1-aryl-4-nitropyrazoles of the formula (I)

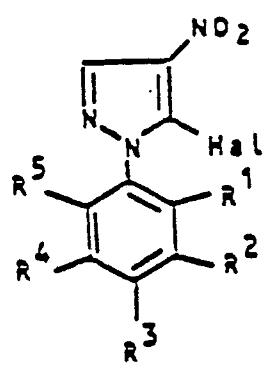

(I)

in which

Hal represents halogen,

$R^1$ and $R^3$ independently of one another represent cyano, nitro or halogen, or represent in each case straight-chain or branched alkyl, alkoxy or alkoxycarbonyl with in each case 1 to 4 carbon atoms in the alkyl part, or represent in each case straight-chain or branched halogenoalkyl or halogenoalkoxy with in each case 1 to 4 carbon atoms and 1 to 9 identical or different halogen atoms, or represent a radical $-S(O)_n-R^6$ and

$R^2$, $R^4$ and $R^5$ independently of one another and independently of $R^1$ and $R^3$ represent the same radicals as $R^1$ and $R^3$ and additionally represent hydrogen,

wherein

$R^6$ represents amino, or in each case represents straight-chain or branched alkyl, alkylamino, dialkylamino or halogenoalkyl with in each case 1 to 4 carbon atoms in the individual alkyl parts and, in the case of halogenoalkyl, with 1 to 9 identical or different halogen atoms and

n represents the number 0, 1 or 2,

characterised in that

(a) 1-arylpyrazoles of the general formula (II)

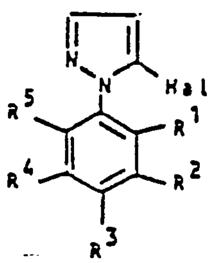

(II)

in which

$R^1$, $R^2$, $R^3$, $R^4$, $R^5$ and Hal have the above-mentioned meaning,

are reacted with nitric acid, if appropriate in the presence of a diluent and if appropriate in the presence of a catalyst, or

(b) 5-amino-4-nitropyrazoles of the formula (III)

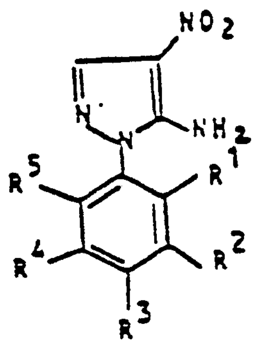

(III)

in which

$R^1$, $R^2$, $R^3$, $R^4$, $R^5$ and Hal have the above-mentioned meaning,

are reacted with nitrite compounds of the formula (IV)

R-O-N=O (IV)

in which

R represents hydrogen, an alkali metal cation or alkyl,

and a haloform of the formula (V)

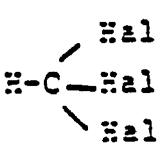

(V)

in which

Hal has the above-mentioned meaning,

if appropriate in the presence of a catalyst, or in that (c) the 1-aryl-4-nitropyrazoles obtainable according to process (a) or (b), of the formula (I)

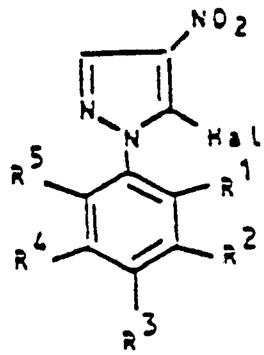

(I)

in which
$R^1$, $R^2$, $R^3$, $R^4$, $R^5$ and Hal have the above-mentioned meaning,
are reacted with halides of the formula (VI)

$M^{\oplus}$ $Hal'^{\ominus}$ (VI)

in which
$M^{\oplus}$ represents one equivalent of a metal cation or an optionally substituted ammonium ion and
$Hal'^{\ominus}$ represents halogen, but is other than Hal,
if appropriate in the presence of a diluent and if appropriate in the presence of a phase transfer catalyst.

4. Herbicidal agents, characterised in that they contain at least one 1-aryl-4-nitropyrazole of the formula (I) according to Claims 1 to 3.

5. Method of combating weeds, characterised in that 1-aryl-4-nitropyrazoles of the formula (1) according to Claims 1 to 3 are applied to the weeds and/or their environment.

6. Use of 1-aryl-4-nitropyrazoles of the formula (I) according to Claims 1 to 3 for combating weeds.

7. Process for the preparation of herbicidal agents, characterised in that 1-aryl-4-nitropyrazoles of the formula (I) according to Claims 1 to 3 are mixed with extenders and/or surface-active substances.

8. 1-Arylpyrazoles of the formula (II)

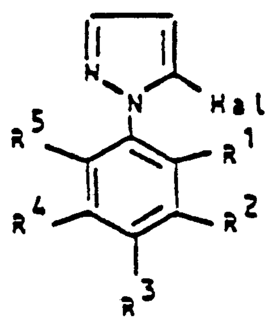

(II)

in which
Hal, $R^1$, $R^2$, $R^3$, $R^4$ and $R^5$ have the meanings mentioned in Claim 1.

9. Process for the preparation of 1-arylpyrazoles of the formula (II)

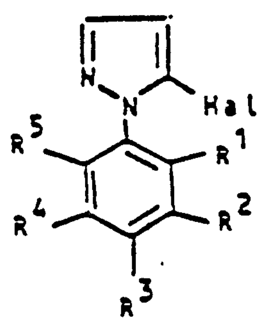

(II)

in which
Hal, $R^1$, $R^2$, $R^3$, $R^4$ and $R^5$ have the meanings mentioned in Claim 3, characterised in that alkoxymethylenemalonates of the formula (VII)

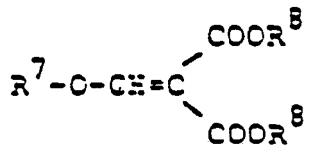

(VII)

in which

$R^7$ and $R^8$ independently of one another in each case represent alkyl, in particular methyl or ethyl, are initially reacted with phenylhydrazines of the formula (VIII)

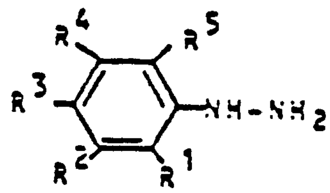

(VIII)

in which

$R^1$, $R^2$, $R^3$, $R^4$ and $R^5$ have the above-mentioned meaning,

in a first stage, if appropriate in the presence of a diluent, at temperatures between +10°C and +80°C and the pyrazolecarboxylic acid esters thus obtainable, of the formula (IX)

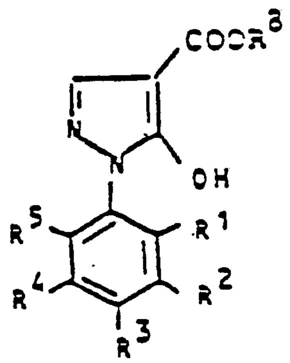

(IX)

in which

$R^1$, $R^2$, $R^3$, $R^4$, $R^5$ and $R^8$ have the above-mentioned meaning,

are decarboxylated in a 2nd stage, if appropriate in the presence of a diluent and if appropriate in the presence of a base, at temperatures between 30°C and 70°C and the pyrazolinones thus obtainable, of the formula (X)

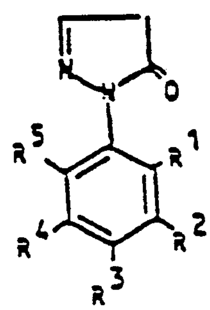

(X)

in which

$R^1$, $R^2$, $R^3$, $R^4$ and $R^5$ have the above-mentioned meaning,

are then reacted with halogenating agents in a 3rd stage.

**Revendications**

1. 1-aryl-4-nitropyrazoles de formule générale (I)

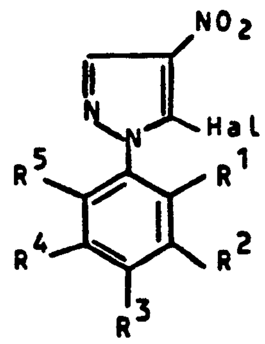

(I)

dans laquelle

Hal représente un halogène,

$R^1$ et $R^3$ représentent indépendamment l'un de l'autre un groupe cyano, nitro, un halogène ou un groupe alkyle, alcoxy ou alcoxycarbonyle, chaque fois à chaîne linéaire ou ramifiée et chaque fois avec 1 à 4 atomes de carbone dans la partie alkyle, un halogéno-alkyle ou un halogéno-alcoxy, chaque fois à chaîne linéaire ou ramifiée, chaque fois avec 1 à 4 atomes de carbone et 1 à 9 atomes d'halogène identiques ou différents, ou un reste $-S(O)_n-R^6$, et

$R^2$, $R^4$ et $R^5$ représentent indépendamment l'un de l'autre et indépendamment de $R^1$ et $R^3$ les mêmes restes que $R^1$ et $R^3$ et de plus l'hydrogène, tandis que

$R^6$ représente le groupe amino, les groupes alkyle, alkylamino, dialkylamino ou halogéno-alkyle, chaque fois à chaîne linéaire ou ramifiée, avec chaque fois 1 à 4 atomes de carbone dans les parties alkyle individuelles et, dans le cas d'un halogéno-alkyle, avec 1 à 9 atomes d'halogène identiques ou différents, et

n représente le nombre 0, 1 ou 2.

2. 1-aryl-4-nitropyrazoles de formule (I) selon la revendication 1,

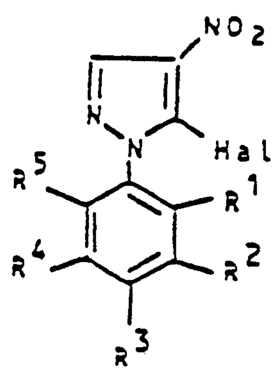

(I)

dans laquelle

Hal représente le fluor, le chlore, le brome ou l'iode,

$R^1$ et $R^3$ représentent indépendamment l'un de l'autre un groupe cyano, nitro, le fluor, le chlore, le brome, l'iode, un groupe méthyle, éthyle, n- et i-propyle, n-, i-, s- et t-butyle, méthoxy, éthoxy, méthoxycarbonyle, éthoxycarbonyle, trifluorométhyle, trichlorométhyle, dichlorofluorométhyle, difluorochlorométhyle, chlorométhyle, dichlorométhyle, difluorochlorométhyle, pentafluoroéthyle, tétrafluoroéthyle, trifluorochloroéthyle, trifluoroéthyle, difluorodichloroéthyle, trifluorodichloroéthyle, pentachloroéthyle, trifluorométhoxy, trichlorométhoxy, dichlorofluorométhoxy, difluorochlorométhoxy, chlorométhoxy, dichlorométhoxy, difluorométhoxy, pentafluoroéthoxy, tétrafluoroéthoxy, trifluorochloroéthoxy, trifluoroéthoxy, difluorodichloroéthoxy, trifluorodichloroéthoxy, pentachloroéthoxy ou un reste $-S(O)_n-R^6$,

et

$R^2$, $R^4$ et $R^5$ représentent indépendamment l'un de l'autre et indépendamment de $R^1$ et $R^3$ les mêmes restes que $R^1$ et $R^3$ et, de plus, l'hydrogène, tandis que

$R^6$ représente un groupe amino, méthylamino, éthylamino, diméthylamino, diéthylamino, fluorodichlorométhyle, difluorométhyle, tétrafluoroéthyle, trichloroéthyle, trifluorométhyle, méthyle ou éthyle

et

n représente le nombre 0, 1 ou 2.

3. Procédé pour la fabrication des 1-aryl-4-nitropyrazoles de formule (I)

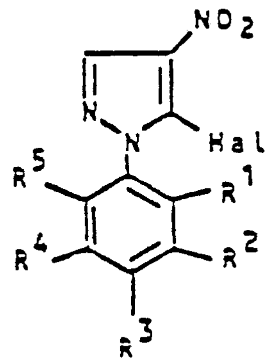

(I)

dans laquelle

Hal représente un halogène,

$R^1$ et $R^3$ représentent indépendamment l'un de l'autre un groupe cyano, nitro, un halogène ou un groupe alkyle, alcoxy ou alcoxycarbonyle, chaque fois à chaîne linéaire ou ramifiée et chaque fois avec 1 à 4 atomes de carbone dans la partie alkyle, un halogéno-alkyle ou un halogène-alcoxy, chaque fois à chaîne linéaire ou ramifiée, chaque fois avec 1 à 4 atomes de carbone et 1 à 9 atomes d'halogène identiques ou différents, ou un reste $-S(O)_n-R^6$, et

$R^2$, $R^4$ et $R^5$ représentent indépendamment l'un de l'autre et indépendamment de $R^1$ et $R^3$ les mêmes restes que $R^1$ et $R^3$ et de plus l'hydrogène, tandis que

$R^6$ représente le groupe amino, les groupes alkyle, alkylamino, dialkylamino ou halogéno-alkyle, chaque fois à chaîne linéaire ou ramifiée, avec chaque fois 1 à 4 atomes de carbone dans les parties alkyle individuelles et, dans le cas d'un halogéno-alkyle, avec 1 à 9 atomes d'halogène identiques ou différents, et

n représente le nombre 0, 1 ou 2,

caractérisé en ce que l'on fait réagir

(a) des 1-arylpyrazoles de formule générale (II)

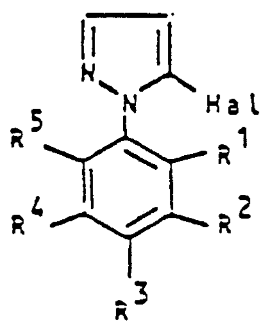

(II)

dans laquelle

$R^1$, $R^2$, $R^3$, $R^4$, $R^5$ et Hal ont les significations décrites ci-dessus,

avec de l'acide nitrique, éventuellement en présence d'un agent de dilution, ainsi qu'éventuellement en présence d'un catalyseur, ou bien

(b) des 5-amino-4-nitropyrazoles de formule (III),

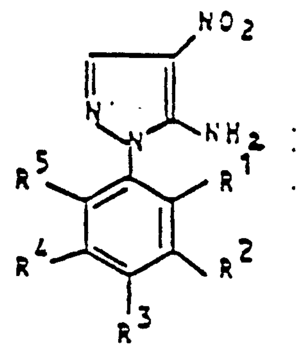

(III)

dans laquelle

$R^1$, $R^2$, $R^3$, $R^4$, $R^5$ et Hal ont les significations décrites ci-dessus,

avec des nitrites de formule (IV),

R-O-N=O (IV)

dans laquelle

R représente l'hydrogène, un cation de métal alcalin ou un groupe alkyle,

et un halogénoforme de formule (V),

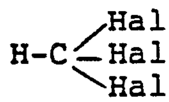

(V)

dans laquelle

Hal a la signification décrite ci-dessus, éventuellement en présence d'un catalyseur, ou bien

(c) les 1-aryl-4-nitropyrazoles pouvant être obtenus selon le procédé (a) ou (b) et répondant à la formule (I)

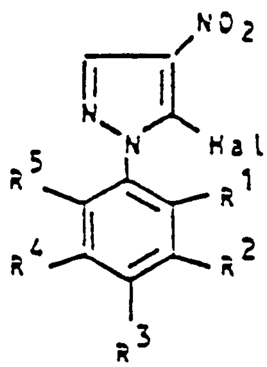

(I)

dans laquelle

$R^1$, $R^2$, $R^3$, $R^4$, $R^5$ et Hal ont les significations décrites ci-dessus,

avec des halogénures de formule (VI),

$M^{\oplus}$ Hal'$^{\ominus}$ (VI)

dans laquelle

$M^{\oplus}$ représente un équivalent d'un cation métallique ou un ion ammonium éventuellement substitué et

Hal'$^{\ominus}$ représente un halogène, mais est différent de Hal,

éventuellement en présence d'un agent de dilution et éventuellement en présence d'un catalyseur de transfert de phase.

4. Agent herbicide, caractérisé en ce qu'il contient au moins un 1-aryl-4-nitropyrazole de formule (I) selon les revendications 1 à 3.

5. Procédé pour la lutte contre les mauvaises herbes, caractérisé en ce que l'on applique les 1-aryl-4-nitropyrazoles de formule (I) selon les revendications 1 à 3 sur les mauvaises herbes et/ou leur habitat.

6. Utilisation des 1-aryl-4-nitropyrazoles de formule (I) selon les revendications 1 à 3 pour la lutte contre les mauvaises herbes.

7. Procédé pour la fabrication d'agents herbicides, caractérisés en ce que l'on mélange les 1-aryl-4-nitropyrazoles de formule (I) selon les revendications 1 à 3 avec des agents de dilution et/ou des substances tensio-actives.

8. 1-arylpyrazoles de formule (II)

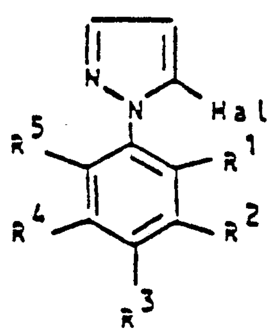

(II)

dans laquelle

Hal, $R^1$, $R^2$, $R^3$, $R^4$ et $R^5$ ont les significations indiquées dans la revendication 1.

9. Procédé pour la fabrication des 1-arylpyrazoles de formule (II)

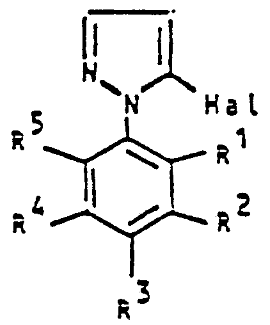

(II)

dans laquelle

Hal, $R^1$, $R^2$, $R^3$, $R^4$ et $R^5$ ont les significations indiquées dans la revendication 3,

caractérisé en ce que l'on fait réagir, tout d'abord, en une première étape, éventuellement en présence d'un agent de dilution à des températures dans l'intervalle de +10°C à +80°C, des esters alcoxyméthylène-maloniques de formule (VII)

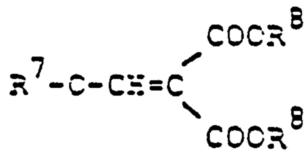

(VII)

dans laquelle

$R^7$ et $R^8$ représentent indépendamment l'un de l'autre un groupe alkyle, en particulier méthyle ou éthyle, avec des phénylhydrazines de formule (VIII),

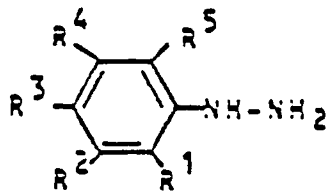

(VIII)

dans laquelle

$R^1$, $R^2$, $R^3$, $R^4$ et $R^5$ ont les significations décrites ci-dessus,

et que les esters d'acide pyrazol-carboxylique ainsi obtenables, de formule (IX)

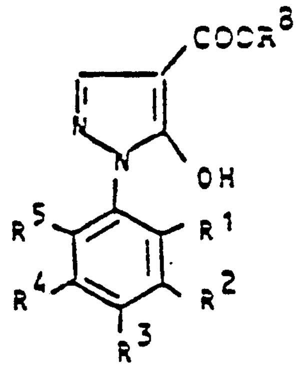

(IX)

dans laquelle

$R^1$, $R^2$, $R^3$, $R^4$, $R^5$ et $R^8$ ont les significations décrites ci-dessus,

sont décarboxylés en une deuxième étape, éventuellement en présence d'un agent de dilution et éventuellement en présence d'une base à des températures comprises entre 30°C et 70°C, et, que, en une troisième étape, on fait réagir les pyrazolinones ainsi obtenables de formule (X)

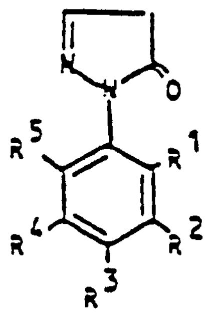

(X)

dans laquelle

$R^1$, $R^2$, $R^3$, $R^4$ et $R^5$ ont les significations décrites ci-dessus, avec des agents d'halogénation.